(19) Europäisches Patentamt
European Patent Office
Office européen des brevets

(11) Veröffentlichungsnummer : **0 500 493 A1**

(12) # EUROPÄISCHE PATENTANMELDUNG

(21) Anmeldenummer : **92810096.5**

(22) Anmeldetag : **11.02.92**

(51) Int. Cl.$^5$ : **C08G 65/40,** C08L 63/00, C07D 333/22

(30) Priorität : **20.02.91 CH 511/91**

(43) Veröffentlichungstag der Anmeldung : **26.08.92 Patentblatt 92/35**

(84) Benannte Vertragsstaaten : **BE DE ES FR GB IT NL SE**

(71) Anmelder : **CIBA-GEIGY AG**
**Klybeckstrasse 141**
**CH-4002 Basel (CH)**

(72) Erfinder : **Pfaendner, Rudolf, Dr.**
**Sackgasse 3**
**W-6149 Rimbach/Odenwald 1 (DE)**
Erfinder : **Wolf, Jean-Pierre, Dr.**
**257, chemin de la motta**
**CH-1791 Courtaman (CH)**
Erfinder : **Kainmüller, Thomas, Dr.**
**Am Kümmelberg 1**
**W-6145 Lindenfels 1 (DE)**
Erfinder : **Kramer, Andreas, Dr.**
**Bundtels 3**
**CH-3186 Düdingen (CH)**
Erfinder : **Hoffmann, Kurt, Dr.**
**Heidenbergstrasse 15**
**W-6147 Lautertal 2 (DE)**
Erfinder : **Stockinger, Friedrich**
**Au Fernotz**
**CH-1784 Courtepin (CH)**

(54) **Neue Polyarylenetherketone.**

(57) Polymere enthaltend 1 - 100 mol% eines wiederkehrenden Strukturelements der Formel (Ia) oder (Ib)

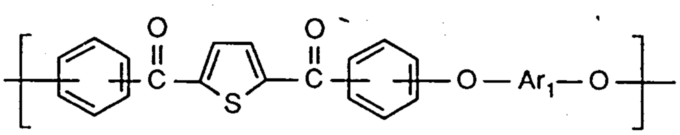 (Ia),

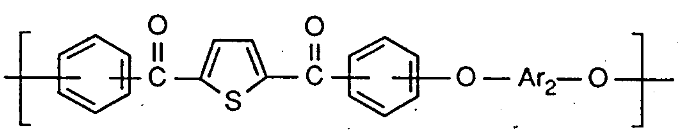 (Ib)

und 99 - 0 mol% eines wiederkehrenden Strukturelements der Formel (II)

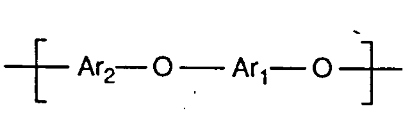 (II),

worin $Ar_1$ für einen oder mehrere Reste der Formeln (IIIa) - (IIIi) steht

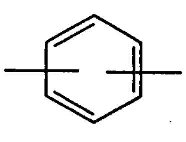 (IIIa), 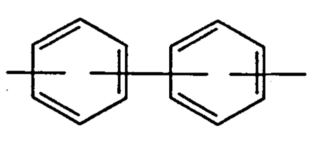 (IIIb), 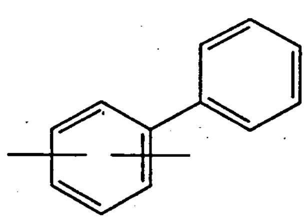 (IIIc),

EP 0 500 493 A1

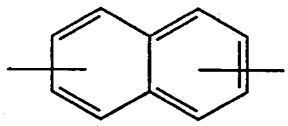 (IIId), 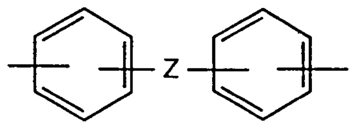 (IIIe),

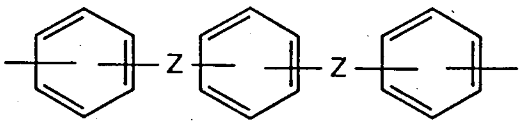 (IIIf),

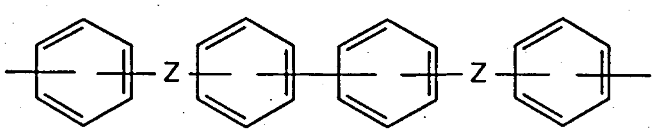 (IIIg),

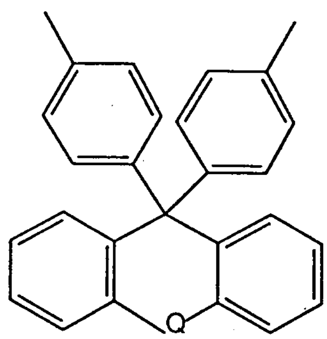 (IIIh), 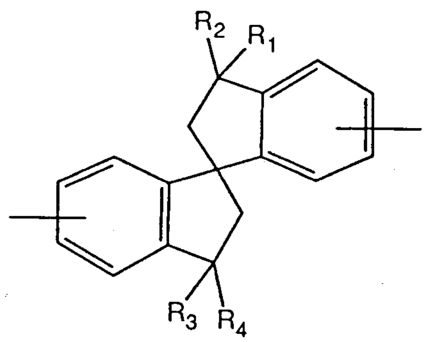 (IIIi),

worin Z $-CH_2-$, $-C(CH_3)_2-$, $-C(CH_3)(C_6H_5)-$, $-C(CF_3)_2-$, -S-, -O-, -SO-, $-SO_2-$ oder -CO-bedeutet, Q für eine direkte Bindung, $-CH_2-$, -O- oder -CO- steht, die Reste $R_1$ bis $R_4$ unabhängig voneinander Wasserstoff oder ($C_1$-$C_4$)-Alkyl bedeuten, $Ar_2$ für einen oder mehrere Reste der Formeln (IVa) - (IVe) steht,

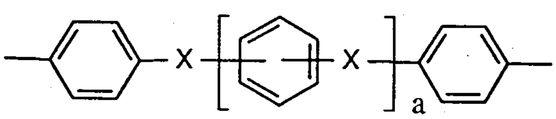 (IVa),

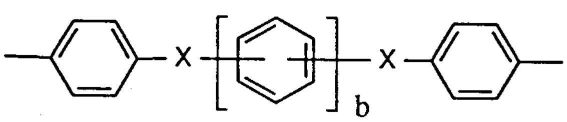 (IVb),

worin X -SO-, $-SO_2-$ oder -CO- bedeutet, a die Zahl 0 oder 1 und b die Zahl 2 oder 3 ist, worin die aromatischen Ringe der Formeln (Ia), (Ib) und (IVa) - (IVe) unsubstituiert oder durch eine oder mehrere ($C_1$-$C_4$)-Alkylgruppen, ($C_1$-$C_4$)-Alkoxygruppen oder ($C_6$-$C_{14}$)-Aryloxygruppen substituiert sind und
worin die aromatischen Ringe der Formeln (IIIa) - (IIIi) unsubstituiert oder durch eine oder mehrere ($C_1$-$C_4$)-Alkylgruppen, ($C_1$-$C_4$)-Alkoxygruppen, ($C_6$-$C_{14}$)-Aryloxygruppen oder Halogenatome substituiert sind,
besitzen eine hohe thermische Stabilität und eignen sich zur Herstellung von Formkörpern, Folien oder Fasern,
als Matrixharze, Klebstoffe oder Überzugsmittel oder als Zusätze zu anderen Polymeren.

Die vorliegende Erfindung betrifft Polymere enthaltend Bis-(benzoyl)-thiophen-Einheiten, ein Verfahren zu deren Herstellung sowie deren Verwendung als technische Werkstoffe.

Polyarylenetherketone sind thermoplastische Konstruktionswerkstoffe mit sehr guten thermischen und mechanischen Eigenschaften, die für Anwendungen eingesetzt werden, bei denen eine hohe Temperaturbeständigkeit gefordert wird.

In gewissen Anwendungsbereichen, beispielsweise wenn eine hohe Löslichkeit der Polymeren gefragt ist, finden Polymere mit heterozyklischen Grundbausteinen Verwendung.
So werden z.B. in J. Pol. Sci., Part A: Pol. Chem. 28, 3179 (1989) Polyarylenethersulfone mit Thiophen-Einheiten beschrieben, die in polaren aprotischen Lösungsmitteln, wie Dimethylformamid und N-Methylpyrrolidon, gut löslich sind, -jedoch nur relativ niedrige Molekulargewichte und unzureichende thermische Stabilitäten aufweisen.

Demgegenüber sind die in J. Pol. Sci., Part A: Pol. Chem. 27, 2417 (1989) offenbarten Polyketone mit Thiophen-Einheiten kristalline Polymere, die in gebräuchlichen organischen Lösungsmitteln nur wenig löslich oder unlöslich sind und eine gute Chemikalienbeständigkeit aufweisen. Die thermische Stabilität dieser Polyketone ist jedoch völlig unzureichend für eine Verarbeitung bei Temperaturen weit oberhalb des Schmelzpunkts, die beispielsweise beim Extrusions- oder Spritzguss-Verfahren erforderlich sind.

Es wurden nun Polyarylenetherketone auf der Basis von Bis(benzoyl)thiophen-Einheiten hergestellt, die sich durch eine sehr hohe Stabilität gegenüber thermischer Zersetzung auszeichnen. Je, nach Struktur der verwendeten Monomeren wurden dabei lösliche Polymere erhalten, die eine hohe Löslichkeit in gebräuchlichen organischen Lösungsmitteln aufweisen, oder (teil-)kristalline Polymere, die lediglich in konzentrierter Schwefelsäure löslich sind.

Gegenstand der vorliegenden Erfindung sind Polymere enthaltend 1 - 100 mol% eines wiederkehrenden Strukturelements der Formel (Ia) oder (Ib)

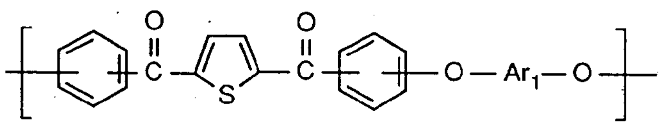 (Ia),

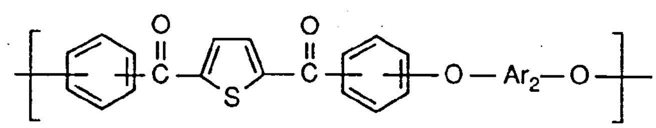 (Ib)

und 99 - 0 mol% eines wiederkehrenden Strukturelements der Formel (II)

$$-\!\left[ Ar_2 - O - Ar_1 - O \right]\!- \quad \text{(II)},$$

worin $Ar_1$ für einen oder mehrere Reste der Formeln (IIIa) - (IIIi) steht

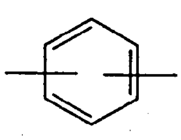 (IIIa), 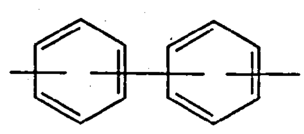 (IIIb), 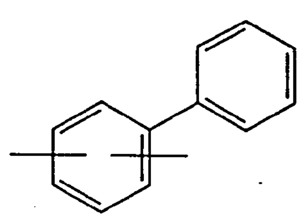 (IIIc),

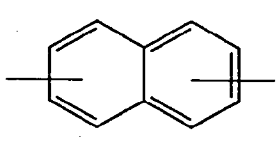 (IIId), 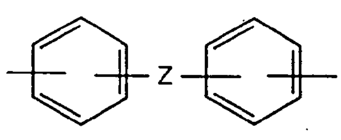 (IIIe),

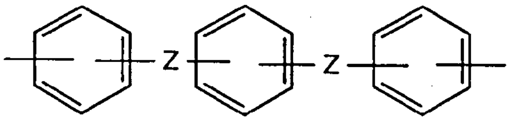 (IIIf),

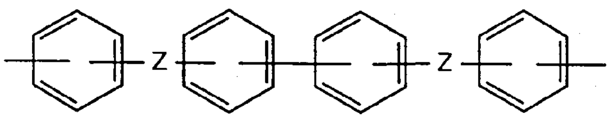 (IIIg),

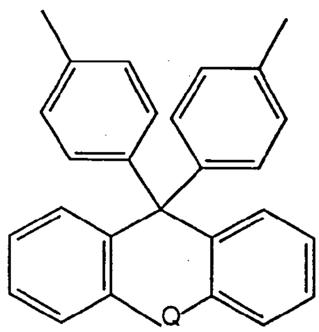 (IIIh), 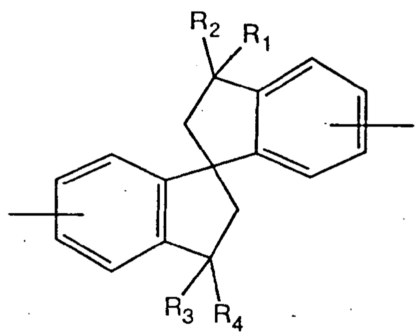 (IIIi),

worin Z $-CH_2-$, $-C(CH_3)_2-$, $-C(CH_3)(C_6H_5)-$, $-C(CF_3)_2-$, -S-, -O-, -SO-, $-SO_2-$ oder -CO-bedeutet, Q für eine direkte Bindung, $-CH_2-$, -O- oder -CO- steht, die Reste $R_1$ bis $R_4$ unabhängig voneinander Wasserstoff oder $(C_1-C_4)$-Alkyl bedeuten, $Ar_2$ für einen oder mehrere Reste der Formeln (IVa) - (IVe) steht,

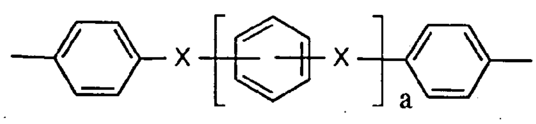 (IVa),

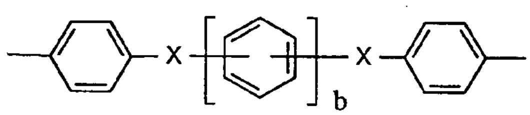 (IVb),

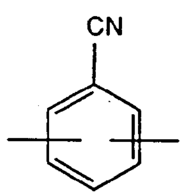 (IVc), 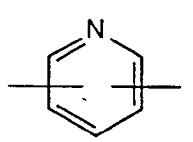 (IVd), 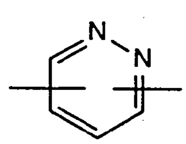 (IVe),

worin X -SO-, $-SO_2-$ oder -CO- bedeutet, a die Zahl 0 oder 1 und b die Zahl 2 oder 3 ist, worin die aromatischen Ringe der Formeln (Ia), (Ib) und (IVa) - (IVe) unsubstituiert oder durch eine oder mehrere $(C_1-C_4)$-Alkylgruppen, $(C_1-C_4)$-Alkoxygruppen oder $(C_6-C_{14})$-Aryloxygruppen substituiert sind und worin die aromatischen Ringe der Formeln (IIIa) - (IIIi) unsubstituiert oder durch eine oder mehrere $(C_1-C_4)$-Alkylgruppen, $(C_1-C_4)$-Alkoxygruppen, $(C_6-C_{14})$-Aryloxygruppen oder Halogenatome substituiert sind.

Vorzugsweise enthalten die erfindungsgemässen Polymeren 10-100 mol% eines wiederkehrenden Strukturelements der Formel (Ia) oder (Ib) und 90-0 mol% eines wiederkehrenden Strukturelements der Formel (II).

Besonders bevorzugte erfindungsgemässe Polymere enthalten 25-100 mol% eines wiederkehrenden Strukturelements der Formel (Ia) oder (Ib) und 75-0 mol% eines wiederkehrenden Strukturelements der Formel (II).

Die aromatischen Ringe der Formeln (Ia), (Ib), (IIIa) = (IIIi) und (IVa) - (IVe) sind vorzugsweise unsubstituiert.

Sind irgendwelche aromatischen Ringe der Formeln (Ia), (Ib), (IIIa) - (IIIi) und (IVa) - (IVe) durch ($C_6$-$C_4$)-Alkylgruppen oder ($C_1$-$C_4$)-Alkoxygruppen substituiert, so handelt es sich dabei um verzweigte oder insbesondere um geradkettige Reste.

Beispiele für solche Alkylgruppen sind Methyl, Ethyl, n-Propyl, Isopropyl, n-Butyl, sek.-Butyl oder tert.-Butyl.

Beispiele für Alkoxygruppen sind Methoxy, Ethoxy, n-Propoxy, Isopropoxy, n-Butoxy oder tert.-Butoxy.

Sind irgendwelche aromatischen Ringe der Formeln (Ia), (Ib), (IIIa) - (IIIi) und (IVa) - (IVe) durch ($C_6$-$C_{14}$)-Aryloxygruppen substituiert, so handelt es sich dabei in der Regel um unsubstituierte Aryloxygruppen. Die Aryloxygruppen können aber ihrerseits auch Substituenten tragen, wie z.B. Alkylgruppen Beispiele für Aryloxygruppen sind Phenyloxy, Naphthyloxy, Biphenyloxy oder Anthryloxy.

Sind die Reste der Formeln (IIIa) - (IIIi) durch Halogenatome substituiert, so handelt es sich dabei um Fluor, Iod oder insbesondere um Chlor oder Brom.

Eine bevorzugte Ausführungsform der vorliegenden Erfindung bilden lösliche Polymere mit einer reduzierten Viskosität von 0,1 bis 2,5 dl/g, bestimmt durch Messung von 1 g Polymeren in 100 ml N-Methylpyrrolidon (NMP), enthaltend 1-100 mol% eines wiederkehrenden Strukturelements der Formel (Ia) oder (Ib) und 99-0 mol% eines wiederkehrenden Strukturelements der Formel (II), dadurch gekennzeichnet, dass mindestens 25 % sämtlicher Reste $Ar_1$ und $Ar_2$ ausgewählt sind aus der Gruppe bestehend aus

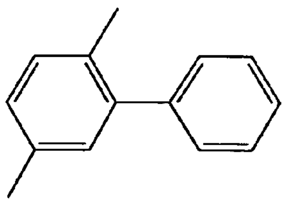 , 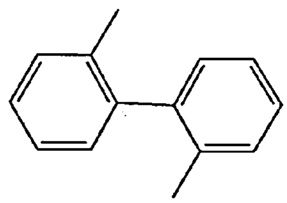 ,

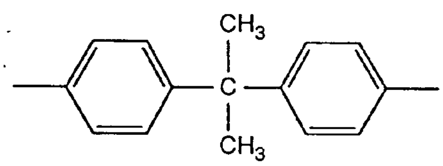 , 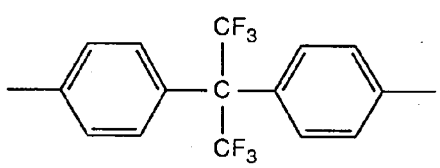 ,

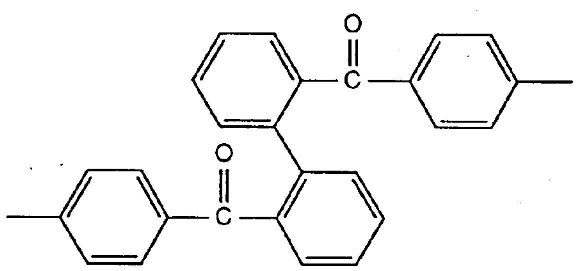

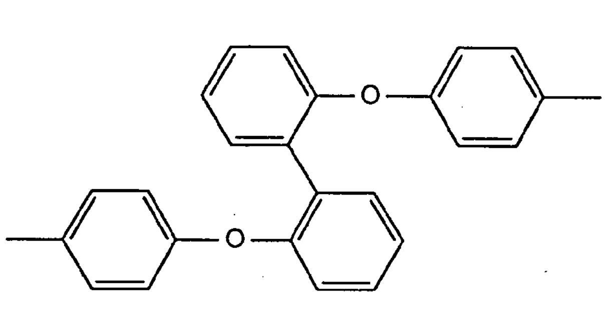 , 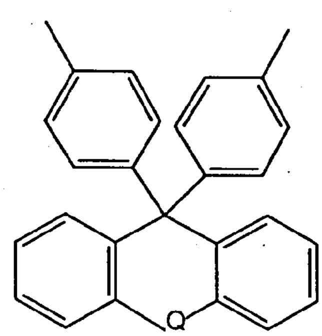

oder

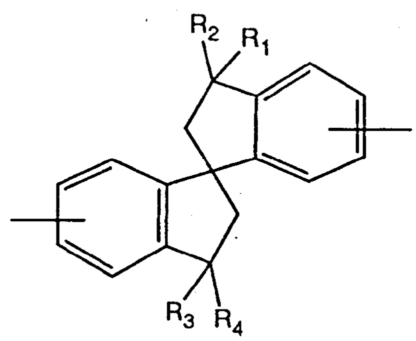,

worin Q, $R_1$, $R_2$, $R_3$ und $R_4$ die oben angegebene Bedeutung haben.

Diese erfindungsgemässen Polyarylenetherketone weisen neben sehr guten thermischen Eigenschaften eine unerwartet hohe löslichkeit in einer Reihe von herkömmlichen organischen Lösungsmitteln auf. Beispiele für geeignete Lösungsmittel sind polare aprotische Lösungsmittel, wie Dimethylsulfoxid, Dimethylacetamid, Dimethylformamid und insbesondere N-Methyl-2-pyrrolidon, zyklische Ether oder Ketone wie Tetrahydrofuran, Dioxan, 1,3-Dioxolan, Cylohexanon und Cyclopentanon, fluorierte oder chlorierte aliphatische und aromatische Kohlenwasserstoffe, wie 1,2-Dichlorethan, Tri- oder Tetrachlorethan, Fluor-dichlorethan und insbesondere Methylenchlorid.

Die Erfindung betrifft daher auch eine Lösung enthaltend etwa 1 bis 75 Gew.%, vorzugsweise 5 bis 50 Gew.%, bezogen auf die Lösung, eines erfindungsgemässen Polyarylenetherketons gelöst in einem organischen Lösungsmittel.

Bevorzugt sind lösliche Polymere, worin mindestens 50 % sämtlicher Reste $Ar_1$ und $Ar_2$ in den Formeln (Ia), (Ib) und (II) ausgewählt sind aus der Gruppe bestehend aus

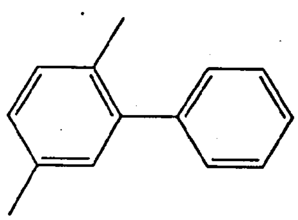, 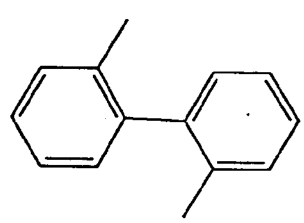,

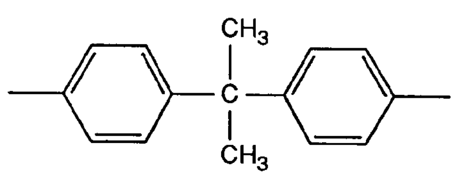, 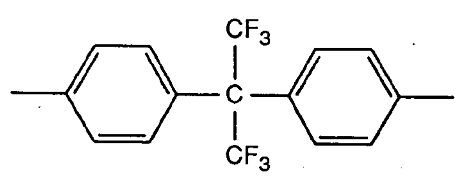,

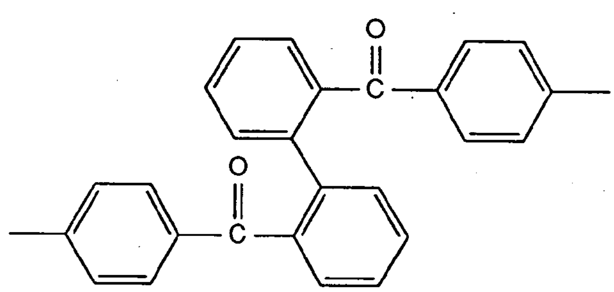,

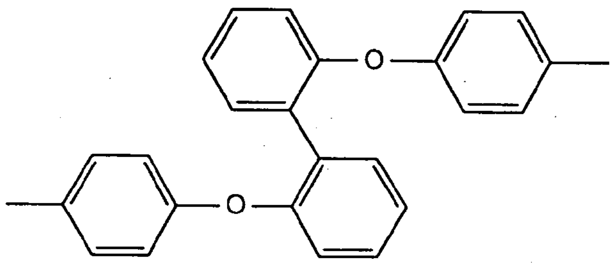

,

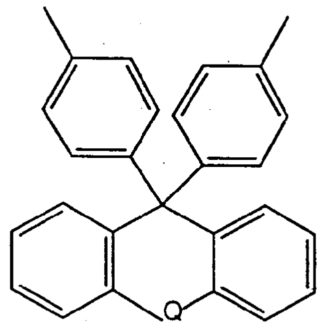

oder

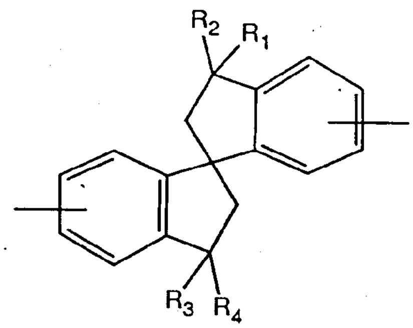

,

worin Q, $R_1$, $R_2$, $R_3$ und $R_4$ die oben angegebene Bedeutung haben.

Vorzugsweise steht $Ar_1$ in den Formeln (Ia) und (II) für

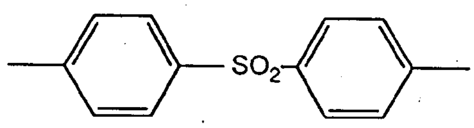

.

$Ar_2$ steht in den Formeln (Ib) und (II) vorzugsweise für

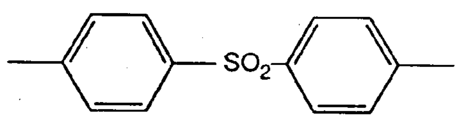

.

Eine weitere bevorzugte Ausführungsform der vorliegenden Erfindung bilden Polymere enthaltend 1-100 mol% eines wiederkehrenden Strukturelements der Formel (Ia) oder (Ib) und 99-0 mol% eines wiederkehrenden Strukturelements der Formel (II), worin $Ar_1$ für einen Rest der Formeln

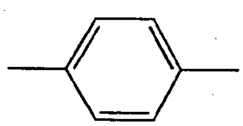

,

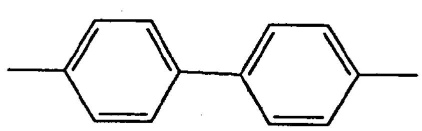

,

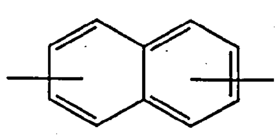

,

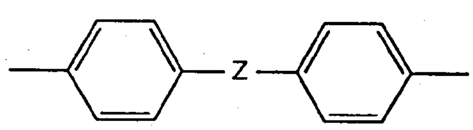

,

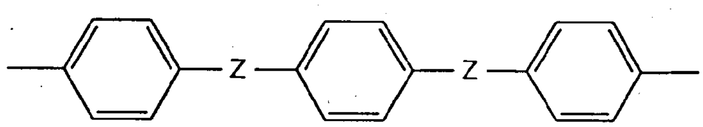,

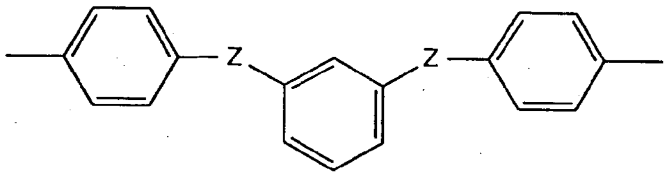

oder

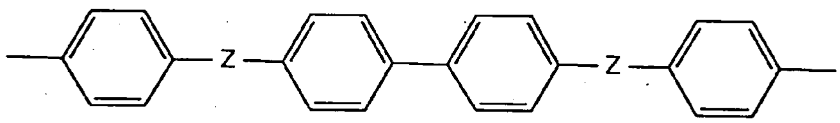

steht und Z $-CH_2-$, $-C(CH_3)_2-$, $-C(CF_3)_2-$, -S-, -O-, -SO-, $-SO_2-$ oder -CO- bedeutet, $Ar_2$ für eine Rest der Formeln (IVa) oder (IVb) steht;

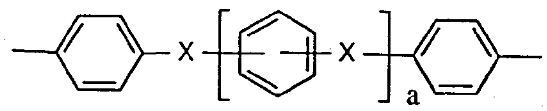 (IVa),

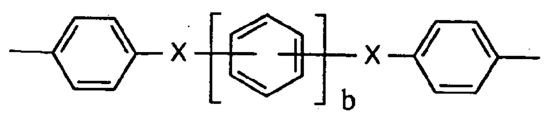 (IVb),

worin X, a und b die oben angegebene Bedeutung haben,
mit der Massgabe, dass weniger als 25 % sämtlicher Reste $Ar_1$ und $Ar_2$ Sulfongruppen enthalten.
Solche Polyarylenetherketone sind (teil-)kristallin und zeichnen sich durch hohe Chemikalienbeständigkeit und hohe Stabilität gegenüber thermischer Zersetzung aus. Sie sind löslich in konzentrierter Schwefelsäure, jedoch unlöslich in gebräuchlichen organischen Lösungsmitteln; wie beispielsweise N-Methylpyrrolidon.

Bevorzugt sind (teil-)kristalline Polymere, worin weniger als 10 % sämtlicher Reste $Ar_1$ und $Ar_2$ Sulfongruppen enthalten.

$Ar_1$ steht in den Formeln (Ia) und (II) vorzugsweise für einen Rest der Formeln

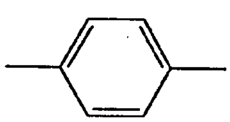, 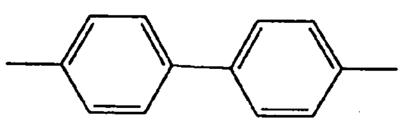,

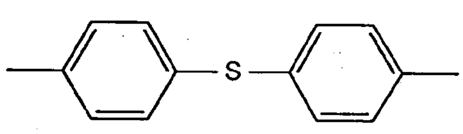, 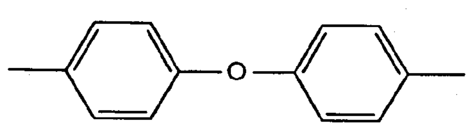,

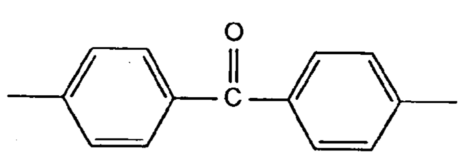,

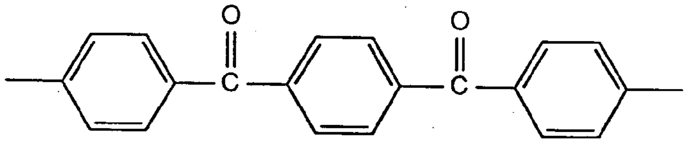

oder

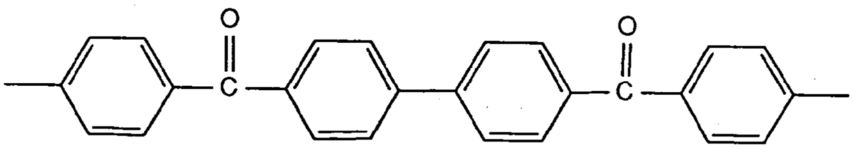

.

Besonders bevorzugt sind (teil-)kristalline Polymere, worin $Ar_1$ in den Formeln (Ia) und

(II) für 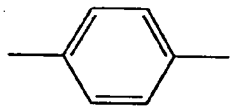 , 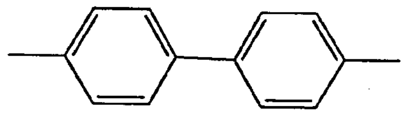

oder

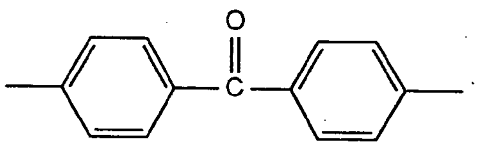

steht.

$Ar_2$ steht in den Formeln (Ib) und (II) vorzugsweise für

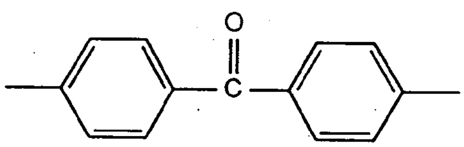

oder

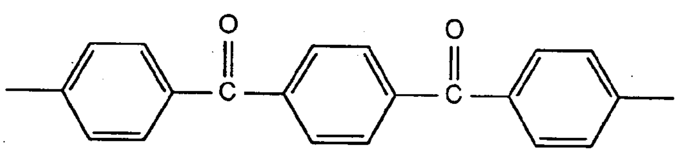

.

Besonders bevorzugt sind (teil-)kristalline Polymere, worin $Ar_2$ in den Formeln (Ib) und

(II) für 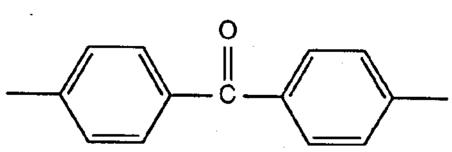

steht.

Die erfindungsgemässen,Polymeren enthaltend 1 - 10 mol% eines wiederkehrenden Strukturelements der Formel (Ia) und 99 - 0 mol% eines wiederkehrenden Strukturelements der Formel (II) können beispielsweise hergestellt werden, indem man eine oder mehrere Dihydroxyverbindungen der Formel (V)

HO—$Ar_1$—OH (V),

worin $Ar_1$ die oben angegebene Bedeutung hat,
mit einer Dihalogenverbindung der Formel (VI)

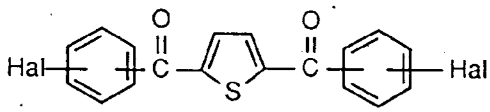 (VI),

worin die aromatischen Ringe unsubstituiert oder durch eine oder mehrere ($C_1$-$C_4$)-Alkylgruppen, ($C_1$-$C_4$)-Alkoxygruppen oder ($C_6$-$C_{14}$)-Aryloxygruppen substituiert sind und Hal für ein Halogenatom, insbesondere Fluor oder Chlor, steht,
oder mit einem Gemisch aus einer Dihalogenverbindung der Formel (VI) und einer darin bis zu 99 mol% enthaltenen Dihalogenverbindung der Formel (VII)

Hal—$Ar_2$—Hal (VII),

worin $Ar_2$ die obenangegebene Bedeutung hat und Hal für ein Halogenatom steht, in an sich bekannter Weise in Gegenwart alkalischer Katalysatoren in einem polaren aprotischen Lösungsmittel polykondensiert.

Die erfindungsgemässen Polymeren enthaltend 1 - 100 mol% eines wiederkehrenden Strukturelements der Formel (Ib) und 99 - 0 mol% eines wiederkehrenden Strukturelements der Formel (II) können hergestellt werden, z.B. indem man man eine Dihalogenverbindung der Formel (VII)

Hal—$Ar_2$—Hal (VII),

worin $Ar_2$ die oben angegebene Bedeutung hat und Hal für ein Halogenatom, insbesondere Fluor oder Chlor, steht,
mit einer Dihydroxyverbindung der Formel (VIII)

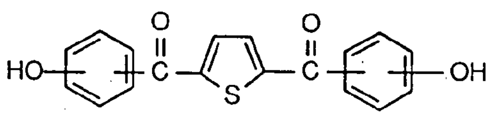 (VIII),

worin die aromatischen Ringe unsubstituiert oder durch eine oder mehrere ($C_1$-$C_4$)-Alkylgruppen, ($C_1$-$C_4$)-Alkoxygruppen oder ($C_6$-$C_{14}$)-Aryloxygruppen substituiert sind, oder mit einem Gemisch aus einer Dihydroxyverbindung der Formel (VIII) und einer in bis zu 99 mol% enthaltenen Dihydroxyverbindung der Formel (V)

HO—$Ar_1$—OH (V),

worin $Ar_1$ die obenangegebene Bedeutung hat, in an sich bekannter Weise in Gegenwart alkalischer Katalysatoren in einem polaren aprotischen Lösungsmittel polykondensiert.

Anstelle der Dihydroxyverbindungen der Formeln (V) oder (VIII) können in an sich bekannter Weise auch die entsprechenden Alkali- oder Erdalkalisalze eingesetzt werden.

Üblicherweise werden bei der Polykondensationsreaktion Dihalogenverbindung(en) und Dihydroxyverbindung(en) in etwa äquimolaren Mengen eingesetzt. Unter etwa äquimolaren Mengen versteht man in diesem Zusammenhang ein Verhältnis von 0,8- 1,2 mol Dihalogenverbindung zu 1,2-0,8 mol Dihydroxyverbindung. Bevorzugt ist ein Verhältnis von 0,9- 1,1 mol Dihalogenverbindung zu 1,1-0,9 mol Dihydroxyverbindung, besonders bevorzugt ein Verhältnis von 0,95- 1,05 mol Dihalogenverbindung zu 1,05-0,95 mol Dihydroxyverbindung.

Als alkalische Katalysatoren verwendet man in diesem Verfahren in der Regel Alkali- und Erdalkalicarbonate, wie Natrium-, Kalium- oder Calciumcarbonat; doch können auch andere alkalische Reagentien, wie Natriumhydroxid, Kaliumhydroxid oder Calcium-hydroxid, Verwendung finden.

Polare aprotische Lösungsmittel, die beim Verfahren zur Herstellung der erfindungsgemässen Polymeren eingesetzt werden können, sind beispielsweise Dimethylsulfoxid, Dimethylformamid, Dimethylacetamid, Diethylacetamid, Tetramethylharnstoff, N-Methylcaprolactam, N-Methyl-2-pyrrolidon und bevorzugt Diphenylsulfon.

Die Reaktion wird zweckmässig bei erhöhter Temperatur durchgeführt, vorzugsweise bis zur Rückflusstemperatur des Lösungsmittels, also etwa bis 350 °C.

Häufig empfiehlt sich die Mitverwendung eines Schleppmittels, wie z.B. Chlorbenzol, Xylol oder Toluol, um das bei der Umsetzung gebildete Wasser aus dem Reaktionsgemisch azeotrop entfernen zu können.

Die Dihydroxyverbindungen der Förmel (V) ind bekannt und zum Teil im Handel erhältlich.

Geeignete Verbindungen der Formel (V) sind beispielsweise Hydrochinon, Bisphenol-A, B isphenol-F, 4,4'-Dihydroxydiphenylether, 4,4'-Dihydroxydiphenylsulfid, Phenylhydrochinon, 1,5-Dihydroxynaphthalin, 2,6-Di-

hydroxynaphtha1in, 2,7-Dihydroxynaphthalin, 9,9-Bis(4-hydroxyphenyl)fluoren, 2,2'-Dihydroxybiphenyl, 4,4'-Dihydroxybiphenyl, 4,4'-Dihydroxydiphenylsulfon, 6,6'-Dihydroxy-3,3,3'3'-tetramethylspirobiindan, 4,4'-Dihydroxybenzophenon und 1,4-Bis(4-hydroxybenzoyl)benzol.

Die Dihalogenverbindungen der Formel (VII) sind ebenfalls bekannt. Geeignete Verbindungen der Formel (VII) sind beispielsweise 4,4'-Difluorbenzophenon, 4,4'-Dichlorbenzophenon, 1,4-Bis(4-fluorbenzoyl)benzol, 4,4'-Difluordiphenylsulfon und 4,4'-Dichlordiphenylsulfon.

Die Dichlor- und Dibromverbindungen der Formel (VI) sind aus J. Heterocyclic Chem. 16, 1147 (1979) bekannt.

Die Difluor- bzw. Dihydroxyverbindungen der Formel (IX)

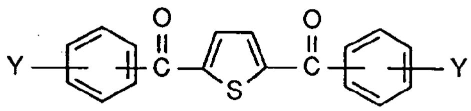 (IX),

worin the aromatischen Ringe unsubstituiert oder durch eine oder mehrere ($C_1$-$C_4$)-Alkylgruppen, ($C_1$-$C_4$)-Alkoxygruppen oder ($C_6$-$C_{14}$)-Aryloxygruppen substituiert sind und Y Fluor oder Hydroxy bedeutet, sind neu und bilden einen weiteren Erfindungsgegenstand. Sie können analog zum für die entsprechenden Dichlor- bzw. Dibromverbindungen beschriebenen Verfahren hergestellt werden, beispielsweise indem man ausgehend von einer Verbindung der Formel (Xa)

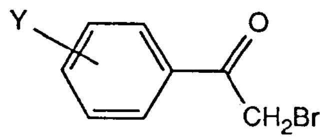 (Xa),

worin der Phenylkerne unsubstituiert oder durch eine oder mehrere ($C_4$-$C_4$)- Alkylgruppen, ($C_1$-$C_4$)-Alkoxygruppen oder ($C_6$-$C_{14}$)-Alkoxygruppen substituiert ist und Y die oben angegebene Bedeutung hat, durch Umsetzung mit Natriumsulfid ein Diketon der Formel(Xb) synthetisiert

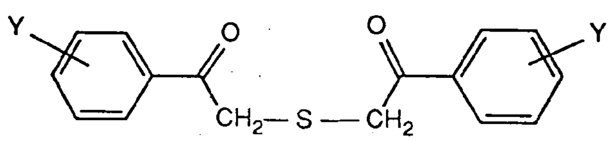 (Xb),

worin die Phenylkerne unsubstituiert oder durch eine oder mehrere ($C_1$-$C_4$)-Alkylgruppen, ($C_1$-$C_4$)-Alkoxygruppen oder ($C_6$-$C_{14}$)-Aryloxygruppen substituiert sind und Y die oben angegebene Bedeutung hat, und das so erhaltene Zwischenprodukt mit Glyoxal in Gegenwart von Basen zu einer Difluor- bzw. Dihydroxyverbindung der Formel (IX) kondensiert.

Beispiele für erfindungsgemässe Verbindungen der Formel (IX) sind 2,5-Bis(2-hydroxybenzoyl)-thiophen, 2,5-Bis(2-fluorbenzoyl)-thiophen, 2,5-Bis(4-hydroxybenzoyl)-thiophen und insbesondere 2,5-Bis(4-fluorbenzoyl)-thiophen.

Besonders hervorzuheben ist die für thiophengruppenhaltige Polymere unerwartet hohe thermische Stabilität der erfindungsgemässen Polyarylenetherketone.

Die erfindungsgemässen Polymeren können in der für Thermoplaste üblichen Weise eingesetzt werden und z.B. zu Formkörpern, Folien oder Fasern verarbeitet werden, oder als Matrixharze, Klebstoffe oder Ueberzugsmitiel oder als Zusätze zu anderen Polymeren verwendet werden.

Vor der Verarbeitung der beispielsweise als Presspulver, Granulat, Schmelze oder insbesondere als Lösung vorliegenden Polymeren können übliche Zusatzstoffe, wie beispielsweise Füllstoffe, Pigmente, Stabilisatoren oder Verstärkungsmittel, wie Kohlenstoff-, Bor-, Metall- oder Glasfasern, zugegeben werden. Die erfindungsgemässen Polymeren können auch zusammen mit anderen Thermoplasten oder Dufluomeren verarbeitet werden.

Vorzugsweise eignen sich die erfindungsgemässen Polyarylenetherketone als Matrixharze für die Herstellung von Faserverbundsystemen, wobei man als Verstärkungsfasern die üblichen bei der Verstärkung technischer Werkstoffe verwendeten Fasern einsetzen kann. Diese können organische oder anorganische Fasern, Naturfasern oder Synthesefasern sein, und als Faserbündel, als orientierte oder nicht-orientierte Fasern oder

als Endlosfasern vorliegen.

Eine bevorzugte Anwendungsmöglichkeit für die erfindungsgemässen löslichen Polymeren besteht in der modifizierung anderer Kunststoffe, insbesondere von Epoxid- und Bismaleinimidharzen. Besondere Bedeutung erlangen solche Systeme als Matrixharze, welche zur Herstellung von Verbundbauteilen zum Einsatz gelangen.

Einen weiteren Erfindungsgegenstand bilden daher Zusammensetzungen enthaltend

a) mindestens ein Epoxidharz,

b) mindestens einen aminischen oder phenolischen Härter und

c) ein lösliches erfindungsgemässes Polyarylenetherketon.

Vorzugsweise enthalten die härtbaren Zusammensetzungen

a) 20-80 Gew.-% eines Epoxidharzes,

b) 5-50 Gew.-% eines aminischen Härters und

c) 2-40 Gew.-% eines löslichen erfindungsgemässen Polyarylenetherketons.

Besonders bevorzugt sind Zusammensetzungen enthaltend als Komponente a) ein Epoxidharz ausgewählt aus der Gruppe bestehend aus Bisphenol-A-diglycidylether, Bisphenol-F-diglycidylether, Bisphenol-S-diglycidylether, N,N,O-Triglycidylp-aminophenol, N,N,O-Triglycidyl-m-aminophenol und N,N,N',N'-Tetraglycidyldiaminodiphenylmethan und als Komponente b) einen aminischen Härter ausgewählt aus der Gruppe bestehend aus 3,3'-Diaminodiphenylsulfon, 4,4'-Diaminodiphenylsulfon und 4,4-Diaminodiphenylmethan.

## I. Herstellung von Ausgangsverbindungen

### I.1. Bis(4-fluorbenzoylmethyl)-sulfid

Zu einer Lösung von 108,5 g (0,5 mol) ω-Brom-4-fluoracetophenon in 500 ml Aceton wird unter Eiskühlung eine Lösung von 59,9 g (0,25 mol) Natriumsulfid in 250 ml Wasser so zugetropft, dass die Innentemperatur nicht über 5 °C steigt. Nach beendeter Zugabe wird über Nacht bei Raumtemperatur gerührt. Die Suspension wird dann auf 0 °C gekühlt und filtriert, wobei ein weisses Pulver vom Schmelzpunkt 104- 105,5 °C erhalten wird. Ausbeute: 69 g (90 %)

Elementaranalyse berechnet für $C_{16}H_{12}F_2O_2S$:

| | | | | | | |
|---|---|---|---|---|---|---|
| Ber. = | C: | 62,74 | H: | 3,95 | S: | 10,47 |
| Gef. = | C: | 62,52 | H: | 3,94 | S: | 10,44 |

### I.2. 2,5-Bis(4-fluorbenzoyl)-thiophen

Eine Lösung von 74 g (0,352 mol) Glyoxal (trimer, Dihydrat) in 3,71 Methanol wird 1 h unter Rückfluss gekocht und in der Siedehitze mit 269,5 g (0,88 mol) Bis(4-fluorbenzoylmethyl)-sulfid versetzt. Zu der so erhaltenen Lösung wird bei 65 °C eine Lösung von 14,7 g Natriummethylat in 700 ml Methanol zugetropft. Die Suspension wird unter Rühren auf Raumtemperatur und anschliessend mit einem Eisbad auf 5 °C gekühlt und filtriert. Es wird ein weisses Pulver vom Schmelzpunkt 185-187 °C erhalten. Ausbeute: 263 g (91 %)

Elementaranalyse berechnet für $C_{18}H_{10}F_2O_2S$:

| | | | | | | |
|---|---|---|---|---|---|---|
| Ber. = | C: | 65,85 | H: | 3,07 | S: | 9,76 |
| Gef. = | C: | 65,74 | H: | 3,12 | S: | 9.93 |

## II. Polymerbeispiele

### Allgemeine Vorschrift zur Herstellung der Polyarylenetherketone:

Ein Gemisch aus einem Diphenol, wasserfreiem Kaliumcarbonat, Diphenylsulfon und Xylol wird in einem Planschliffkolben mit Rührer unter Stickstoff bis auf eine Badtemperatur von 200 °C erhitzt, wobei eine Xylol/Wasser-Mischung azeotrop abdestilliert. Der Destillationsvorgang wird nach ca. 1,5 h durch kurzzeitiges (ca.

10 min) Anlegen eines Vakuums von ca. 1 mbar abgeschlossen.
Danach wird unter Stickstoff 2,5-Bis(4-fluorbenzoyl)thiophen zur Reaktionsmischung gegeben. Anschliessend wird die Temperatur stufenweise erhöht, wobei die Reaktionsmischung mit fortschreitender Reaktionszeit eine deutliche Viskositätszunahme zeigt. Nach kurzem Abkühlen wird die Reaktionsmischung dem Kolben entnommen und nach Erstarren pulverisiert. Zur Isolierung des Polymeren wird das Gemisch mit Wasser unter Zusatz von Essigsäure, anschliessend mehrfach mit Wasser und schliesslich mit einem Wasser/Aceton-Gemisch (20 Vol.% Wasser, 80 Vol.% Aceton) extrahiert. Das so gereinigte Polymere wird danach im Vakuumtrockenschrank bis zu einer Temperatur von 240 °C getrocknet.

Auf diese Weise werden die löslichen Polarylenyetherketone der Beispiele 1-11 und die (teil-)kristallinen Polyarylenetherketone der Beispiele 12-14 synthetisiert. Die Zusammensetzungen und Eigenschaften der erhaltenen Polymeren sind in den Tabellen 1 und 2 zusammengefasst.

Tabelle 1: lösliche Polyarylenetherketone

| Beispiel | Zusammensetzung | Reaktionsbedingungen | red. Visk. [dl/g] | Löslichkeit NMP | Löslichkeit $CH_2Cl_2$ | $T_g$/°C | $T_z$/°C |
|---|---|---|---|---|---|---|---|
| II.1 | 0,1003 mol 4,4'-Dihydroxydiphenylsulfon<br>0,1000 mol 2,5-Bis(4-fluorbenzoyl)thiophen<br>0,1050 mol Kaliumcarbonat | 1 h/225 °C<br>1 h/257 °C<br>2,5 h/283 °C | 0,52 | 25 | 25 | 171 | 531 |
| II.2 | 0,2004 mol 4,4'-Dihydroxydiphenylsulfon<br>0,1000 mol 2,5-Bis(4-fluorbenzoyl)thiophen<br>0,1001 mol 4,4'-Dichlordiphenylsulfon<br>0,2128 mol Kaliumcarbonat | 1 h/228 °C<br>1 h/250 °C<br>3 h/280 °C | 0,47 | 25 | 25 | 197 | 535 |
| II.3 | 0,2004 mol 4,4'-Dihydroxydiphenylsulfon<br>0,0500 mol 2,5-Bis(4-fluorbenzoyl)thiophen<br>0,1500 mol 4,4'-Dichlordiphenylsulfon<br>0,2110 mol Kaliumcarbonat | 1 h/225 °C<br>1 h/253 °C<br>3 h/283 °C | 0,35 | 25 | 25 | 203 | 544 |
| II.4 | 0,2005 mol 4,4'-Dihydroxydiphenylsulfon<br>0,0201 mol 2,5-Bis(4-fluorbenzoyl)thiophen<br>0,1801 mol 4,4'-Dichlordiphenylsulfon<br>0,2122 mol Kaliumcarbonat | 1 h/226 °C<br>1 h/255 °C<br>2,5 h/284 °C | 1,09 | 25 | 25 | 223 | >550 |
| II.5 | 0,2012 mol 4,4'-Dihydroxydiphenylsulfon<br>0,0201 mol 2,5-Bis(4-fluorbenzoyl)thiophen<br>0,1802 mol 4,4'-Dichlordiphenylsulfon<br>0,2119 mol Kaliumcarbonat | 1 h/226 °C<br>1 h/255 °C<br>2 h/281 °C | 0,78 | 25 | 25 | 219 | 545 |
| II.6 | 0,0501 mol 6,6'-Dihydroxy-3,3,3',3'-tetramethylspirobiindan<br>0,0100 mol 2,5-Bis(4-fluorbenzoyl)thiophen<br>0,0349 mol 4,4'-Dichlordiphenylsulfon<br>0,0527 mol Kaliumcarbonat | 1 h/224 °C<br>1 h/255 °C<br>4 h/278 °C | 0,12 | 25 | 25 | 191 | 470 |

Tabelle 1: lösliche Polyarylenetherketone (Fortsetzung)

| Beispiel | Zusammensetzung | Reaktions-bedingungen | red. Visk. [dl/g] | Löslichkeit NMP | Löslichkeit $CH_2Cl_2$ | $T_g$/°C | $T_z$/°C |
|---|---|---|---|---|---|---|---|
| II.7 | 0,0402 mol 4,4'-Dihydroxydiphenylsulfon<br>0,0102 mol 6,6'-Dihydroxy-3,3,3'3'-tetramethylspirobiindan<br>0,0250 mol 2,5-Bis(4-fluorbenzoyl)thiophen<br>0,0252 mol 2,6-Difluorbenzonitril<br>0,0526 mol Kaliumcarbonat | 1 h/226 °C<br>1 h/250 °C<br>3 h/284 °C | 0,27 | 25 | 25 | 200 | 440 |
| II.8 | 0,0501 mol 4,4'-Dihydroxydiphenylsulfon<br>0,0250 mol 2,5-Bis(4-fluorbenzoyl)thiophen<br>0,0250 mol 4,4'-Difluorbenzophenon<br>0,0525 mol Kaliumcarbonat | 1 h/227 °C<br>1 h/254 °C<br>1 h/279 °C | 0,21 | 25 | 20 | 179 | 455 |
| II.9 | 0,0251 mol 4,4'-Dihydroxydiphenylsulfon<br>0,0251 mol 9,9-Bis(4-hydroxyphenyl)fluoren<br>0,0100 mol 2,5-Bis(4-fluorbenzoyl)thiophen<br>0,0401 mol 4,4'-Dichlordiphenylsulfon<br>0,0521 mol Kaliumcarbonat | 1 h/226 °C<br>1 h/248 °C<br>110 min/<br>282 °C | 0,98 | 25 | 20 | 228 | 530 |
| II.10 | 0,0501 mol 2,2-Bis(4-hydroxyphenyl)hexafluorpropan<br>0,0502 mol 2,5-Bis(4-fluorbenzoyl)thiophen<br>0,0528 mol Kaliumcarbonat | 1 h/228 °C<br>1 h/251 °C<br>25 min/278°C | 1,91 | 25 | 15 | 150 | 540 |
| II.11 | 0,0306 mol 2,2'-Dihydroxybiphenyl<br>0,0305 mol 2,5-Bis(4-fluorbenzoyl)thiophen<br>0,0328 mol Kaliumcarbonat | 1 h/223 °C<br>1 h/250 °C<br>85 min/281°C | 1,72 | 25 | 5 | 157 | 515 |

red. Visk.: reduzierte Viskosität, bestimmt durch Messung von 1 g Polymeren in 100 ml N-Methylpyrrolidon (NMP) bei 25 °C;
Löslichkeit: angegeben in Gew.-%
$T_g$: Glasübergangstemperatur, bestimmt durch Differential Scanning Calorimetry (DSC);
$T_z$: Temperatur, bei der der durch Thermogravimetrie (TGA, Heizrate 10 °C/min, unter Stickstoff) bestimmte Gewichtsverlust 5 % beträgt.

Tabelle 2: (teil-)kristalline Polyarylenetherketone

| Beispiel | Zusammensetzung | Reaktions-bedingungen | $T_1$ [°C] | $T_k$ [°C] | $T_m$ [°C] | $T_z$ [°C] |
|---|---|---|---|---|---|---|
| II.12 | 0,0502 mol 4,4'-Dihydroxybiphenyl<br>0,0500 mol 2,5-Bis(4-fluorbenzoyl)thiophen<br>0,0527 mol Kaliumcarbonat | 1 h/236 °C<br>1 h/257 °C<br>1 h/283 °C | 285 | 315 | 359 | 550 |
| II.13 | 0,0501 mol 4,4'-Dihydroxybenzophenon<br>0,0500 mol 2,5-Bis(4-fluorbenzoyl)thiophen<br>0,0528 mol Kaliumcarbonat | 1 h/226 °C<br>1 h/249 °C<br>4 h/278 °C | 228 | 240 | 298/312 | 490 |
| II.14 | 0,0306 mol Hydrochinon<br>0,0306 mol 2,5-Bis(4-fluorbenzoyl)thiophen | 1 h/227 °C<br>1 h/249 °C<br>4 h/283 °C | 203 | 222 | 301 | 540 |

$T_1$: erster nachweisbarer thermischer Übergang
$T_k$: Kristallisationstemperatur beim Abkühlen aus der Schmelze
$T_m$: Schmelztemperatur
$T_z$: Temperatur, bei der der durch Thermogravimetrie (TGA, Heizrate 10 °C/min, unter Stickstoff) bestimmte Gewichtsverlust 5 % beträgt

III. Anwendungsbeispiele

Beispiel III.1.:

Das gemäss Beispiel II.2 synthetisierte Polyarylenetherketonsulfon wird in einer Plattenpresse bei 280 °C unter einem Druck von ca. 25 N/cm$^2$ während 5 min zu einem transparenten Film mit einer Dicke von 0,23 mm verpresst.
An 1 cm breiten Prüfstreifen werden die folgenden mechanischen Eigenschaften (DIN 53455) bestimmt:
Zugfestigkeit: 69, 8 N/mm$^2$
Reissdehnung: 8, 1 %
Elastizitätsmodul: 1460 N/mm$^2$

Beispiele III.2.-III.4.:

Ein gemäss den Beispielen II.2. oder II.5. hergestellter Polyarylenether wird zu 20 bzw. 30 Gewichtsteilen in Methylenchlorid gelöst. Die filtrierte Lösung wird zu einer Mischung aus 50 Gewichtsteilen N,N,N',N'-Tetraglycidyldiaminodiphenylmethan und 50 Gewichtsteilen N,N,O-Triglycidyl-p-aminophenol gegeben. Das Lösungsmittel wird im Vakuum entfernt. Nach Zugabe von 50 Gewichtsteilen p,p-Diaminodiphenylsulfon wird das Gemisch in einer Form während 2 h bei 160 °C und 2 h bei 210 °C ausgehärtet. Aus einer so hergestellten Platte werden Prüfkörper geschniten und die Biegefestigkeit und Randfaserdehnung nach ISO 178 bestimmt. Die Resultate sind in Tabelle 3 angegeben.

Tabelle 3:

| Polyarylenether aus Beispiel | Gewichtsteile Polyarylenether | Biegefestigkeit [N/mm$^2$] | Randfaserdehnung [%] |
|---|---|---|---|
| II.2 | 20 | 151 | 5,4 |
| II.5 | 20 | 153 | 5,4. |
| II.5 | 30 | 162 | 6,2 |

**Patentansprüche**

**1.** Polymere enthaltend 1 - 100 mol% eines wiederkehrenden Strukturelements der Formel (Ia) oder (Ib)

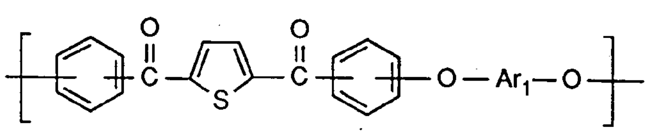 (Ia),

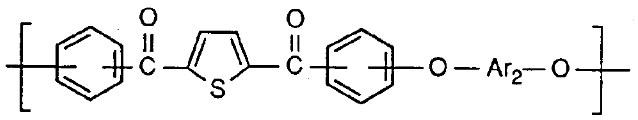 (Ib)

und 99 - 0mol% eines wiederkehrenden Strukturelements der Formel (II)

$$-\!\left[Ar_2-O-Ar_1-O\right]\!- \quad \text{(II)},$$

worin $Ar_1$ für einen oder mehrere Reste der Formeln (IIIa) - (IIIi) steht

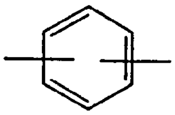 (IIIa), 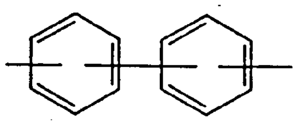 (IIIb), 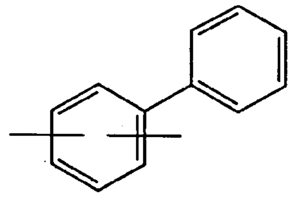 (IIIc),

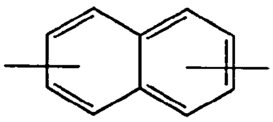 (IIId), 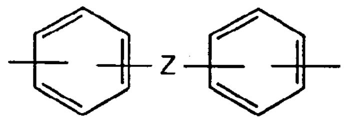 (IIIe),

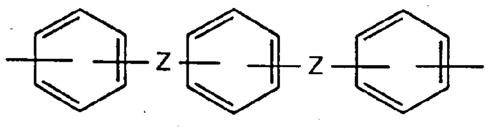 (IIIf),

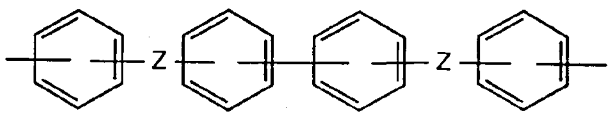 (IIIg),

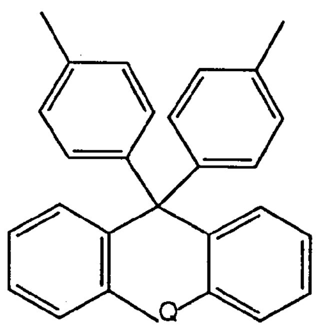 (IIIh), 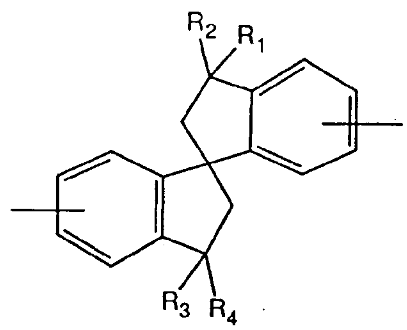 (IIIi),

worin Z $-CH_2-$, $-C(CH_3)_2-$, $-C(CH_3)(C_6H_5)-$, $-C(CF_3)_2-$, -S-, -O-, -SO-, $-SO_2-$ oder -CO-bedeutet, Q für eine direkte Bindung, $-CH_2-$, -O- oder -CO- steht, die Reste $R_1$ bis $R_4$ unabhängig voneinander Wasserstoff oder ($C_1$-$C_4$)-Alkyl bedeuten,

$Ar_2$ für einen oder mehrere Redte der Formeln (IVa) - (IVe) steht,

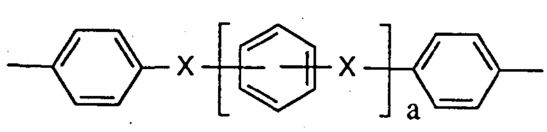 (IVa),

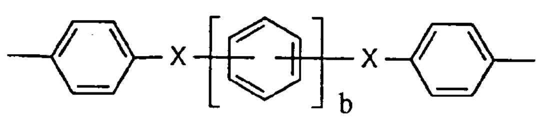 (IVb),

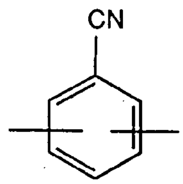 (IVc), 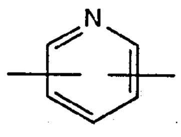 (IVd), 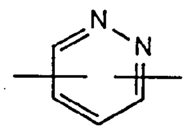 (IVe),

worin X -SO-, $-SO_2-$ oder -CO- bedeutet, a die Zahl 0 oder 1 und b die Zahl 2 oder 3 ist, worin die aromatischen Ringe der Formeln (Ia), (Ib) und (IVa) - (IVe) unsubstituiert oder durch eine oder mehrere ($C_1$-$C_4$)-Alkylgruppen, ($C_1$-$C_4$)-Alkoxygruppen oder ($C_6$-$C_{14}$)-Aryloxygruppen substituiert sind und
worin die aromatischen Ringe der Formeln (IIIa) - (IIIi) unsubstituiert oder durch eine oder mehrere ($C_1$-$C_4$)-Alkylgruppen, ($C_1$-$C_4$)-Alkoxygruppen, ($C_6$-$C_{14}$)-Aryloxygruppen oder Halogenatome substituiert sind.

**2.** Polymere gemäss Anspruch 1 enthaltend 10 - 100 mol% eines wiederkehrenden Strukturelements der Formel (Ia) oder (Ib) und 90 - 0 mol% eines wiederkehrenden Strukturelements der Formel (II).

**3.** Polymere gemäss Anspruch 1, worin die aromatischen Ringe der Formeln (Ia), (Ib), (IIIa) - (IIIi) und (IVa) - (IVe) unsubstituiert sind.

**4.** Polymere gemäss Anspruch 1 mit einer reduzierten Viskosität von 0,1 bis 2,5 dl/g, bestimmt durch Messung von 1 g Polymeren in 100 ml N-Methylpyrrolidon (NMP), dadurch gekennzeichnet, dass mindestens 25 % sämtlicher Reste $Ar_1$ und $Ar_2$ ausgewählt sind aus der Gruppe bestehend aus

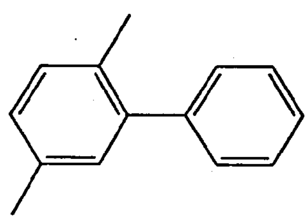 , 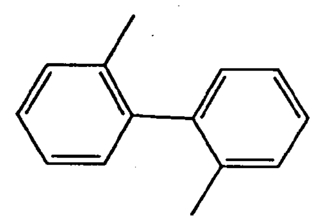 ,

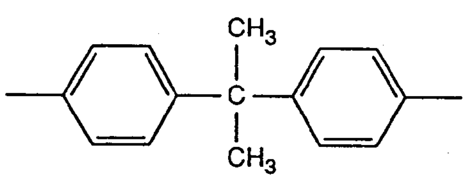 , 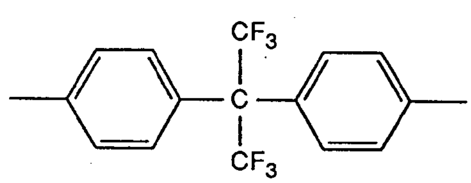 ,

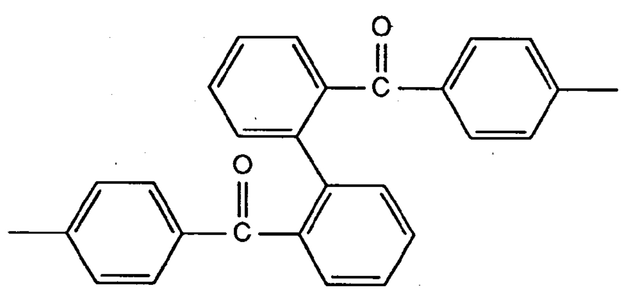

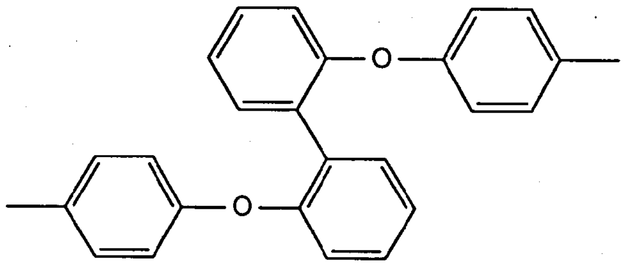 , 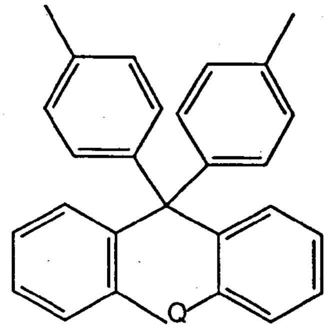 .

oder

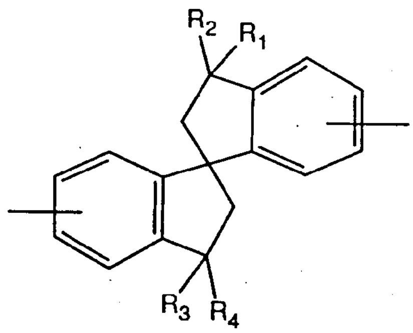 ,

worin Q, $R_1$, $R_2$, $R_3$ und $R_4$ die in Anspruch 1 angegebene Bedeutung haben.

**5.** Lösungen enthaltend etwa 1-75 Gew.%, bezogen auf die gesamte Lösung, eines Polymeren gemäss Anspruch 4 gelöst in einem organischem Lösungsmittel.

**6.** Polymere gemäss Anspruch 4, worin mindestens 50 % sämtlicher Reste $Ar_1$ und $Ar_2$ ausgewählt sind aus der Gruppe bestehend aus

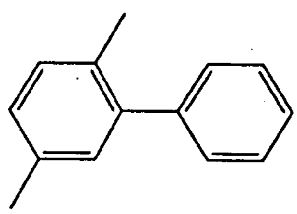 ,

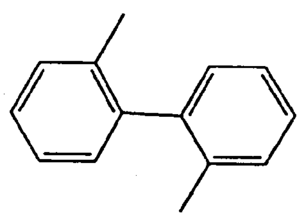 , 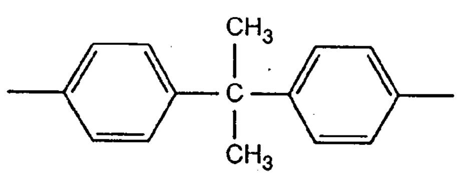 ,

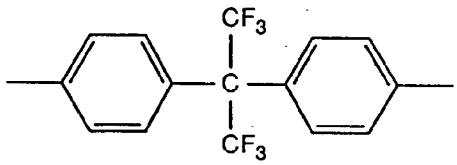,
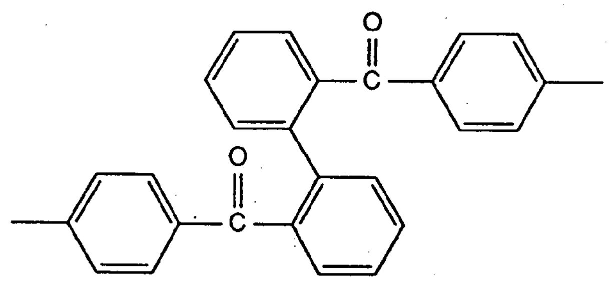,

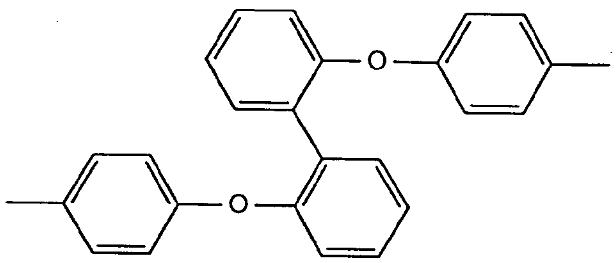,
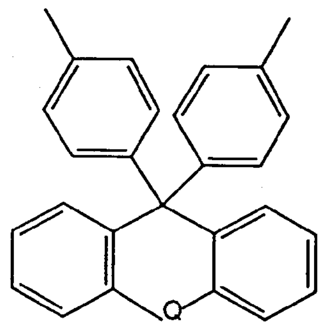,

oder

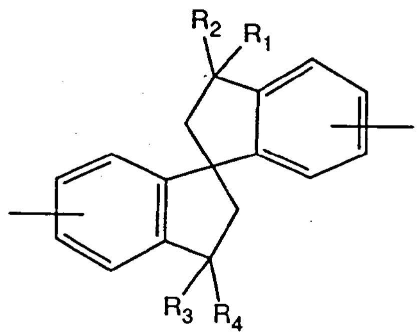,

worin Q, $R_1$, $R_2$, $R_3$ und $R_4$ die in Anspruch 1 angegebene Bedeutung haben.

7. Polymere gemäss Anspruch 4, worin $Ar_1$ für einen Rest der Formel

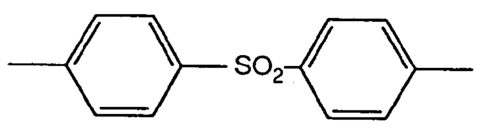

steht.

8. Polymere gemäss Anspruch 4, worin $Ar_2$ für einen Rest der Formel

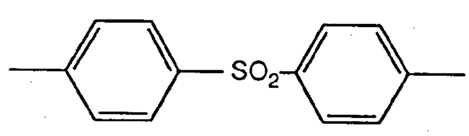

steht.

9. Polymere gemäss Anspruch 1, worin $Ar_1$ für einen Rest der Formeln

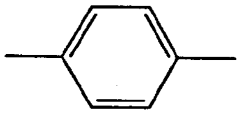 , 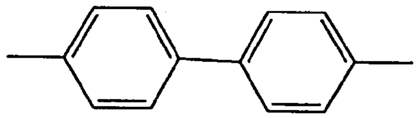 , 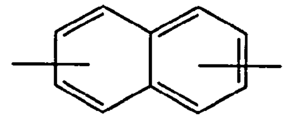 ,

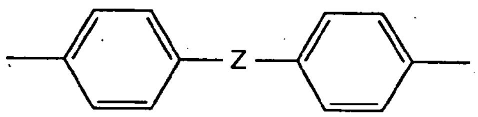 ,

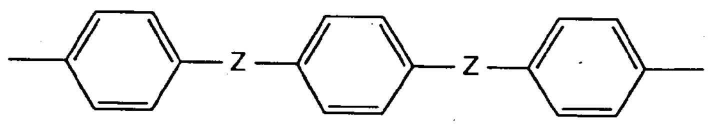 ,

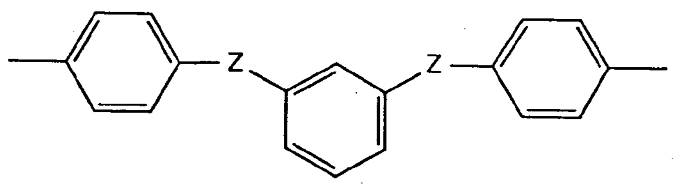

oder

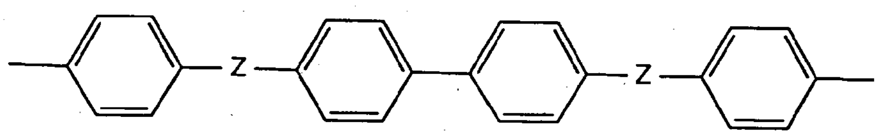 ;

steht und Z $-CH_2-$, $-C(CH_3)_2-$, $-C(CF_3)_2-$, -S-, -O-, -SO-, $-SO_2-$ oder -CO- bedeutet, $Ar_2$ für eine Rest der Formeln (IVa) oder (IVb) steht,

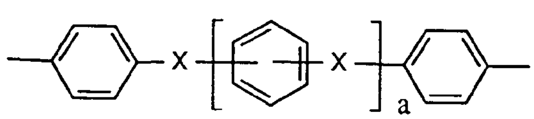 (IVa),

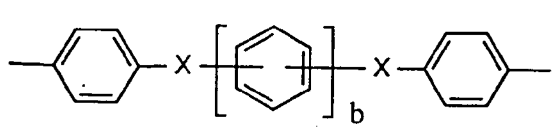 (IVb),

worin X, a und b die in Anspruch 1 angegebene Bedeutung haben, mit der Massgabe, dass weniger als 25 % sämtlicher Reste $Ar_1$ und $Ar_2$ Sulfongruppen enthalten.

10. Polymere gemäss Anspruch 9, dadurch gekennzeichnet, dass weniger als 10 % sämtlicher Reste $Ar_1$ und $Ar_2$ Sulfongruppen enthalten.

11. Polymere gemäss Anspruch 9, worin $Ar_1$ für einen Rest der Formeln

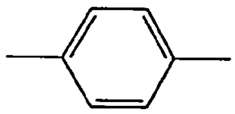 , 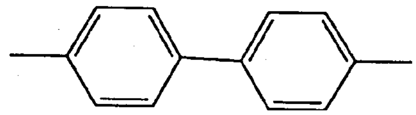 ,

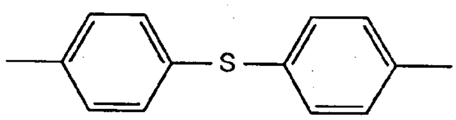 , 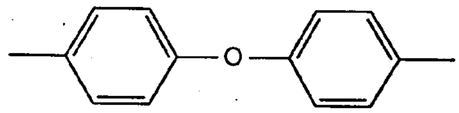 ,

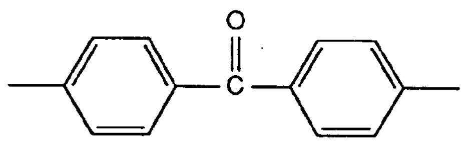 ,

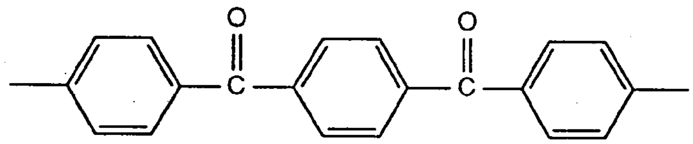

oder

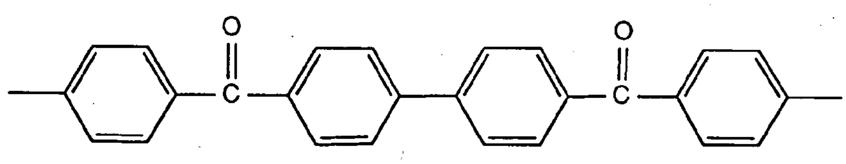

steht.

12. Polymere gemäss Anspruch 9, worin $Ar_1$ für einen Rest der Formeln

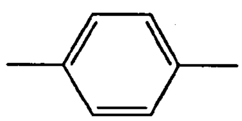 , 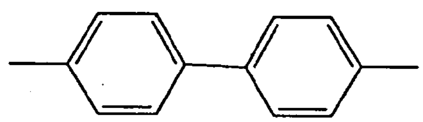

oder

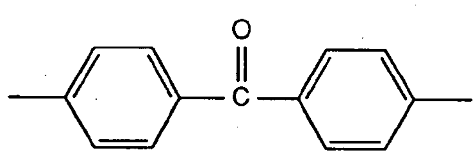

steht.

13. Polymere gemäss Anspruch 9, worin $Ar_2$ für einen Rest der Formeln

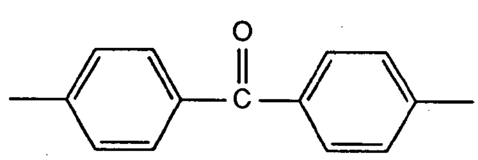

oder

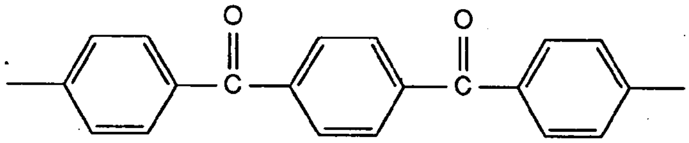

steht.

**14.** Polymere gemäss Anspruch 9, worin $Ar_2$ für

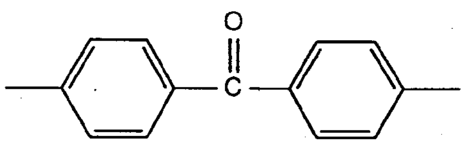

steht.

**15.** Verfahren zur Herstellung von Polymeren gemäss Anspruch 1 enthaltend 1 - 100 mol% eines wiederkehrenden Strukturelements der Formel (Ia) und 99 - 0 mol% eines wiederkehrenden Strukturelements der Formel (II), dadurch gekennzeichnet, dass man eine oder mehrere Dihydroxyverbindungen der Förmel (V)

$$HO—Ar_1—OH \quad (V),$$

worin $Ar_1$ die in Anspruch 1 angegebene Bedeutung hat,
mit einer Dihalogenverbindung der Formel (VI)

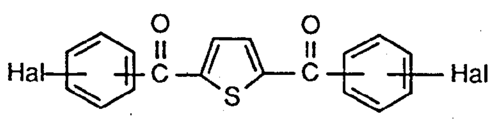 (VI),

worin die aromatischen Ringe unsubstituiert oder durch eine oder mehrere ($C_1$-$C_4$)-Alkylgruppen, ($C_1$-$C_4$)-Alkoxygruppen oder ($C_6$-$C_{14}$)-Aryloxygruppen substituiert sind und Hal für ein Halogenatom, insbesondere Fluor oder Chlor, steht,
oder mit einem Gemisch aus einer Dihalogenverbindung der Formel (VI) und einer darin bis zu 99 mol% enthaltenen Dihalogenverbindung der Formel (VII)

$$Hal—Ar_2—Hal \quad (VII),$$

worin $Ar_2$ die in Anspruch 1 angegebene Bedeutung hat und Hal für ein Halogenatom steht,
in an sich bekannter Weise in Gegenwart alkalischer Katalysatoren in einem polaren aprotischen Lösungsmittel polykondensiert.

**16.** Verfahren zur Herstellung von Polymeren gemäss Anspruch 1 enthaltend 1 - 100 mol% eines wiederkehienden Strukturelements der Formel (Ib) und 99 - 0 mol% eines wiederkehrenden Strukturelements der Formel (II), dadurch gekennzeichnet, dass man eine Dihalogenverbindung der Formel (VII)

$$Hal—Ar_2—Hal \quad (VII),$$

worin $Ar_2$ die in Anspruch 1 angegebene Bedeutung hat und Hal für ein Halogenatom, insbesondere Fluor oder Chlor, steht,
mit einer Dihydroxyverbindung der Formel (VIII)

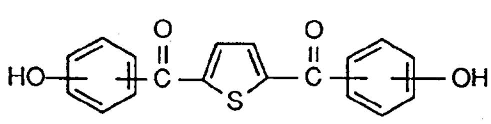 (VIII),

worin die aromatischen Ringe unsubstituiert oder durch eine oder mehrere ($C_1$-$C_4$)-Alkylgruppen, ($C_1$-$C_4$)-Alkoxygruppen oder ($C_6$-$C_{14}$)-Aryloxygruppen substituiert sind, oder mit einem Gemisch aus einer Dihydroxyverbindung der Formel (VIII) und einer darin bis zu 99 mol% enthaltenen Dihydroxyverbindung der Formel (V)

$$HO\ Ar_1—OH\ (V),$$

worin $Ar_1$ die in Anspruch 1 angegebene Bedeutung hat,
in an sich bekannter Weise in Gegenwart alkalischer Katalysatoren in einem polaren aprotischen Lösungsmittel polykondensiert.

**17.** Verbindungen der Formel (IX)

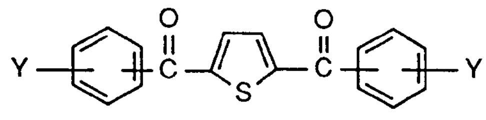 (IX),

worin die aromatischen Ringe unsubstituiert oder durch eine oder mehrere ($C_1$-$C_4$)-Alkylgruppen, ($C_1$-$C_4$)-Alkoxygruppen oder ($C_6$-$C_{14}$)-Aryloxygruppen substituiert sind und Y Fluor oder Hydroxy bedeutet.

**18.** Verwendung der Polymeren gemäss Anspruch 1 zur Herstellung von Formkörpern, Folien oder Fasern, als Matrixharze, Klebstoffe oder Überzugsmittel oder als Zusätze zu anderen Polymeren.

**19.** Verwendung der Polymeren gemäss Anspruch 4 zur Modifizierung von anderen Kunststoffen

**20.** Zusammensetzung enthaltend
a) mindestens ein Epoxidharz,
b) mindestens einen aminischen oder phenolischen Härter und
c) ein Polymer gemäss Anspruch 4.

**21.** Zusammensetzung gemäss Anspruch 20 enthaltend
a) 20-80 Gew.-% eines Epoxidharzes,
b) 5-50 Gew.-% eines aminischen Härters und
c) 2-40 Gew.-% eines Polymers gemäss Anspruch 4.

**22.** Zusammensetzung gemäss Anspruch 20, enthaltend als Komponente a) ein Epoxidharz ausgewählt aus der Gruppe bestehend aus Bisphenol-A-diglycidylether, Bisphenol-F-diglycidylether, Bisphenol-S -diglycidylether, N,N,O-Triglycidyl-p-aminophenol, N,N,O-Triglycidyl-m-aminophenol und N,N,N′,N′-Tetraglycidyldiaminodiphenylmethan und als Komponente b) einen aminischen Härter ausgewählt aus der Gruppe bestehend aus 3,3′-Diaminodiphenylsulfon, 4,4′-Diaminodiphenylsulfon und 4,4-Diaminodiphenylmethan.

**Patentansprüche für folgenden Vertragsstaat: ES**

**1.** Verfahren zur Herstellung von Polymeren enthaltend 1 - 100 mol% eines wiederkehrenden Strukturelements der Formel (Ia) oder (Ib)

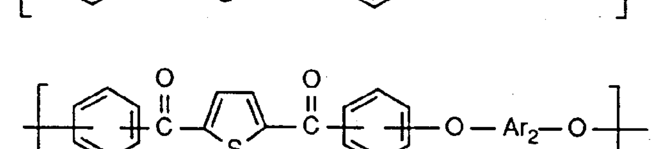 (Ia),

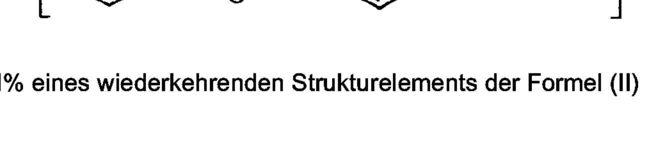 (Ib)

und 99 - 0 mol% eines wiederkehrenden Strukturelements der Formel (II)

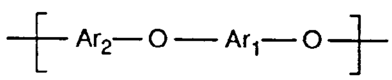 (II),

worin $Ar_1$ für einen oder mehrere Reste der Formeln (IIIa) - (IIIi) steht

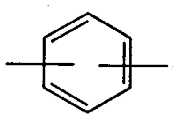 (IIIa), 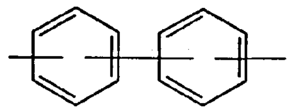 (IIIb), 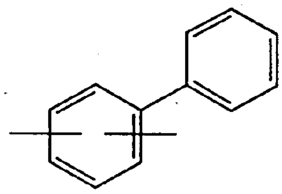 (IIIc),

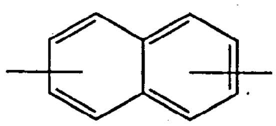 (IIId), 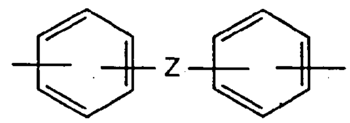 (IIIe),

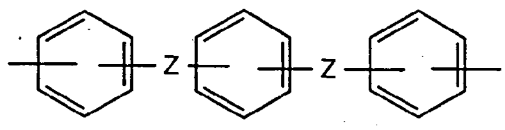 (IIIf),

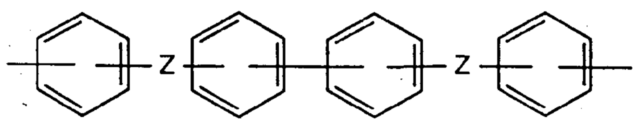 (IIIg),

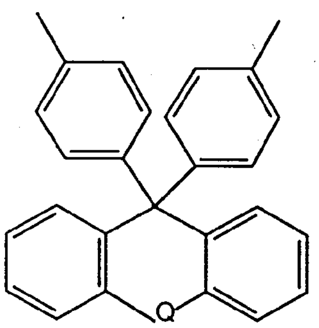 (IIIh), 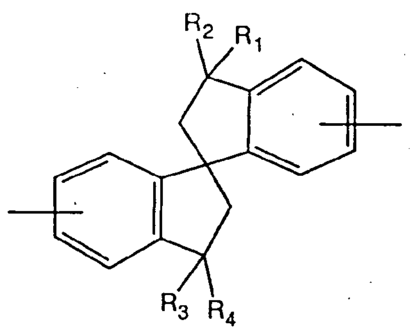 (IIIi),

worin Z $-CH_2-$, $-C(CH_3)_2-$, $-C(CH_3)(C_6H_5)-$, $-C(CF_3)_2-$, -S-, -O-, -SO-, $-SO_2-$ oder -CO-bedeutet, Q für eine direkte Bindung, $-CH_2-$, -O- oder -CO- steht, die Reste $R_1$ bis $R_4$ unabhängig voneinander Wasserstoff oder $(C_1$-$C_4)$-Alkyl bedeuten, $Ar_2$ für einen oder mehrere Reste der Formeln (IVa) - (IVe) steht,

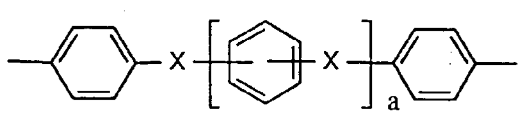 (IVa),

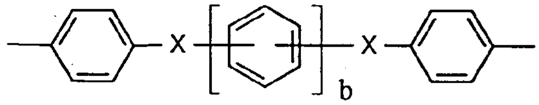 (IVb),

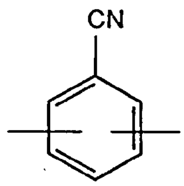 (IVc), 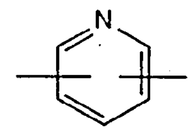 (IVd), 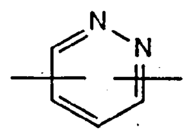 (IVe),

worin X -SO-, $-SO_2-$ oder -CO- bedeutet, a die Zahl 0 oder 1 und b die Zahl 2 oder 3 ist, worin die aromatischen Ringe der Formeln (Ia), (Ib) und (IVa) - (IVe) unsubstituiert oder durch eine oder mehrere ($C_1$-$C_4$)-Alkylgruppen, ($C_1$-$C_4$)-Alkoxygruppen oder ($C_6$-$C_{14}$)-Aryloxygruppen substituiert sind und
worin die aromatischen Ringe der Formeln (IIIa) - (IIIi) unsubstituiert oder durch eine oder mehrere ($C_1$-$C_4$)-Alkylgruppen, ($C_1$-$C_4$)-Alkoxygruppen, ($C_6$-$C_{14}$)-Aryloxygruppen oder Halogenatome substituiert sind, dadurch gekennzeichnet, dass man eine oder mehrere Dihydroxyverbindungen der Formel (V)

$$HO—Ar_1—OH \quad (V),$$

worin $Ar_1$ die obenangegebene Bedeutung hat,
mit einer Dihalogenverbindung der Formel (VI)

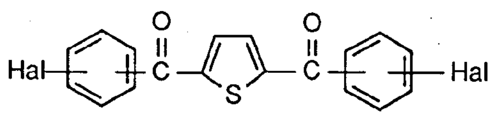 (VI),

worin die aromatischen Ringe unsubstituiert oder durch eine oder mehrere ($C_1$-$C_4$)-Alkylgruppen, ($C_1$-$C_4$)-Alkoxygruppen oder ($C_6$-$C_{14}$)-Aryloxygruppen substituiert sind und Hal für ein Halogenatom, insbesondere Fluor oder Chlor, steht,
oder mit einem Gemisch aus einer Dihalogenverbindung der Formel (VI) und einer darin bis zu 99 mol% enthaltenen Dihalogenverbindung der Formel (VII)

$$Hal—Ar_2—Hal \quad (VII),$$

worin $Ar_2$ die obenangegebene Bedeutung hat und Hal für ein Halogenatom steht, in an sich bekannter Weise in Gegenwart alkalischer Katalysatoren in einem polaren aprotischen Lösungsmittel polykondensiert,
oder dadurch gekennzeichnet, dass man eine Dihalogenverbindung der Formel (VII)

$$Hal—Ar_2—Hal \quad (VII),$$

worin $Ar_2$ die oben angegebene Bedeutung hat und Hal für ein Halogenatom, insbesondere Fluor oder Chlor, steht,
mit einer Dihydroxyverbindung der Formel (VIII)

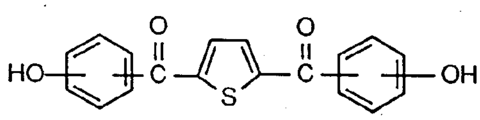 (VIII),

worin die aromatischen Ringe unsubstituiert oder durch eine oder mehrere ($C_1$-$C_4$)-Alkylgruppen, ($C_1$-$C_4$)-Alkoxygruppen oder ($C_6$-$C_{14}$)-Alkoxygruppen substituiert sind, oder mit einem Gemisch aus einer Dihydroxyverbindung der Formel (VIII) und einer darin bis zu 99 mol% enthaltenen Dihydroxyverbindung der Formel (V)

$$HO—Ar_1—OH \quad (V),$$

worin $Ar_1$ die obenangegebene Bedeutung hat,
in an sich bekannter Weise in Gegenwart alkalischer Katalysatoren in einem polaren aprotischen Lösungsmittel polykondensiert.

2. Verfahren gemäss Anspruch 1, dadurch gekennzeichnet, dass die aromatischen Ringe der Formeln (Ia), (Ib), (IIIa) - (IIIi) und (IVa) - (IVe) unsubstituiert sind.

3. Verfahren zur Herstellung von Polymeren gemäss Anspruch 1 mit einer reduzierten Viskosität von 0,1 bis 2,5 dl/g, bestimmt durch Messung von 1 g Polymeren in 100 ml N-Methylpyrrolidon (NMP), dadurch gekennzeichnet, dass mindestens 25 % sämtlicher Reste $Ar_1$ und $Ar_2$ ausgewählt sind aus der Gruppe bestehend aus

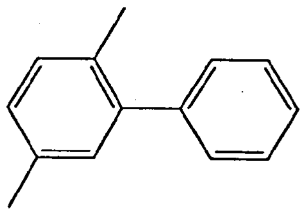 , 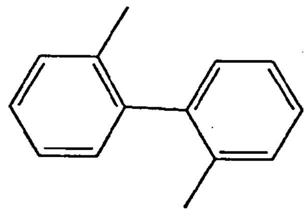 ,

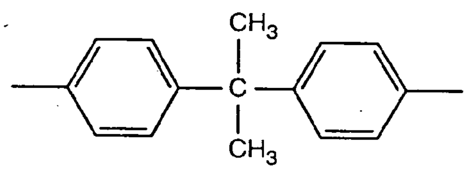 , 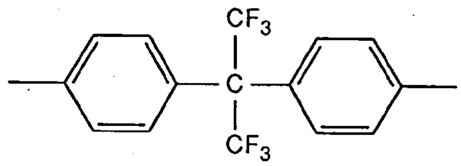 ,

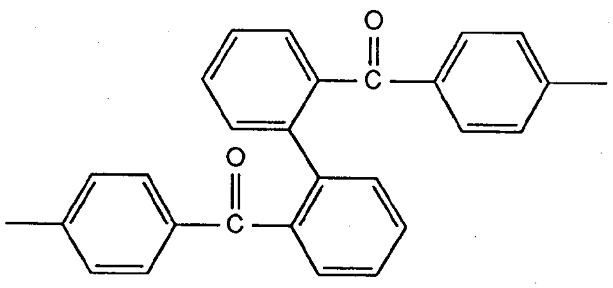

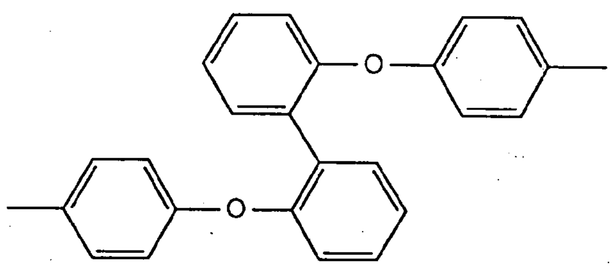 , 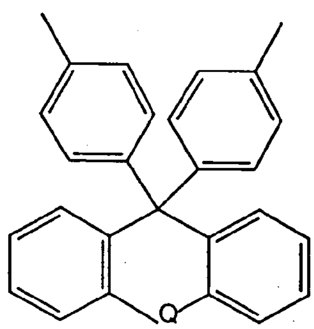 ,

oder

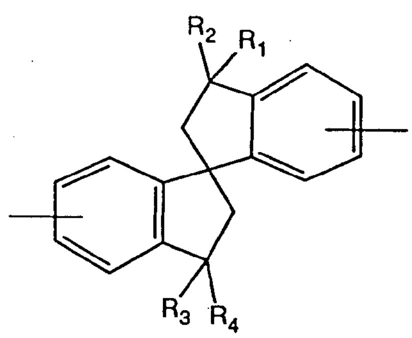 ,

worin Q, $R_1$, $R_2$, $R_3$ und $R_4$ die in Anspruch 1 angegebene Bedeutung haben.

4. Verfahren gemäss Anspruch 3, dadurch gekennzeichnet dass mindestens 50 % sämtlicher Reste $Ar_1$ und $Ar_2$ ausgewählt sind aus der Gruppe bestehend aus

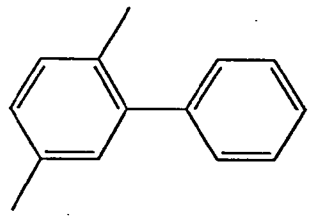

,

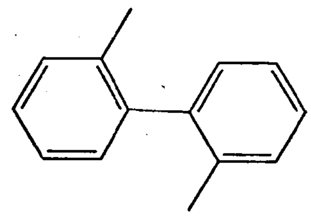

,

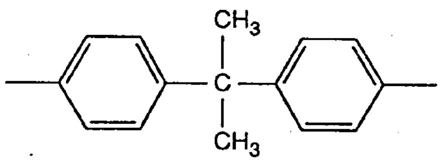

,

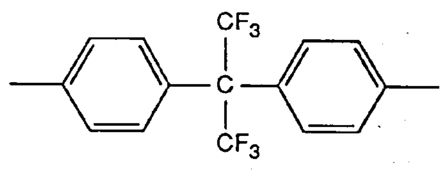

,

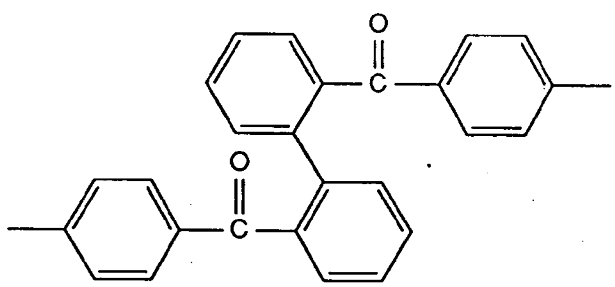

,

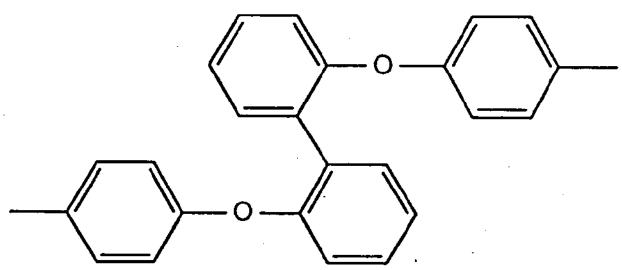

,

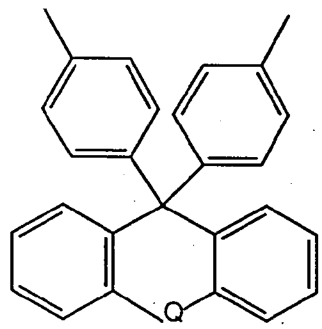

oder

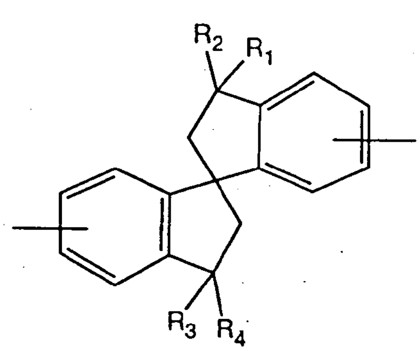

,

worin Q, $R_1$, $R_2$, $R_3$ und $R_4$ die in Anspruch 1 angegebene Bedeutung haben.

**5.** Verfahren gemäss Anspruch 3, dadurch gekennzeichnet, dass $Ar_1$ für einen Rest der Formel

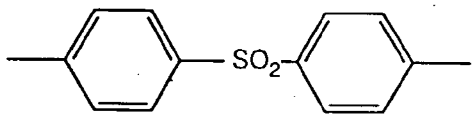

steht.

6. Verfahren gemäss Anspruch 3, dadurch gekennzeichnet, dass$Ar_2$ für einen Rest der Formel

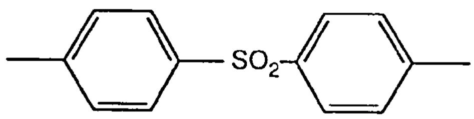

steht.

7. Verfahren gemäss Anspruch 1, dadurch gekennzeichnet, dass $Ar_1$ für einen Rest der Formeln

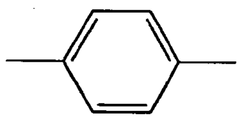 , 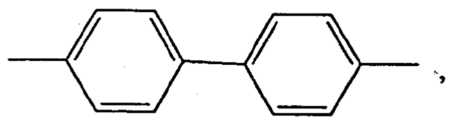 ,

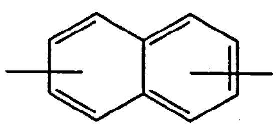 , 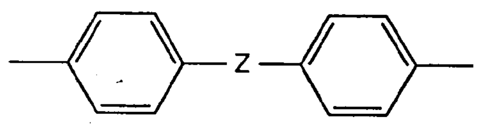 ,

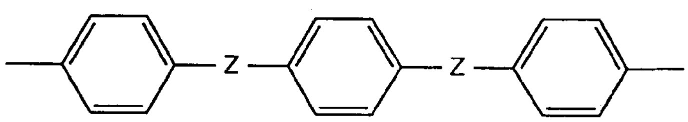 ,

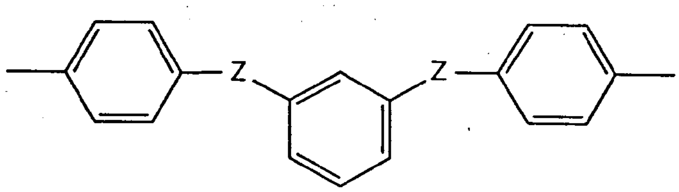

oder

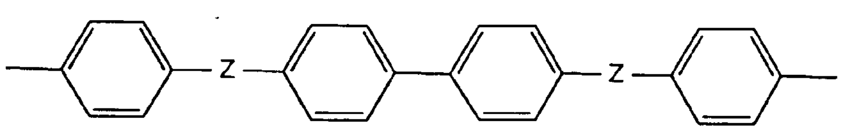

steht und Z-$CH_2$-, -$C(CH_3)_2$-, -$C(CF_3)_2$-, -S-, -O-, -SO-, -$SO_2$- oder -CO- bedeutet, $Ar_2$ für eine Rest der Formeln (IVa) oder (IVb) steht,

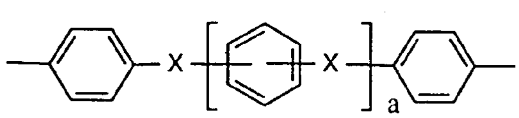 (IVa),

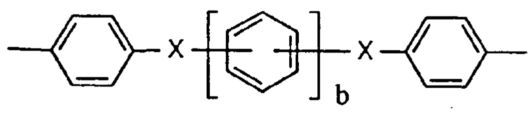 (IVb),

worin X, a und b die in Anspruch 1 angegebene Bedeutung haben,
mit der Massgabe, dass weniger als 25 % sämtlicher Reste $Ar_1$ und $Ar_2$ Sulfongruppen enthalten.

**8.** Verfahren gemäss Anspruch 7, dadurch gekennzeichnet, dassdass weniger als 10 % sämtlicher Reste $Ar_1$ und $Ar_2$ Sulfongruppen enthalten.

**9.** Verfahren gemäss Anspruch 7, dadurch gekennzeichnet, dass $Ar_1$ für einen Rest der Formeln

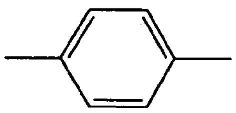 , 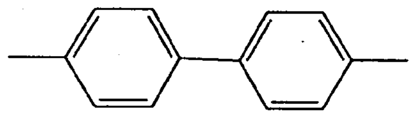 ,

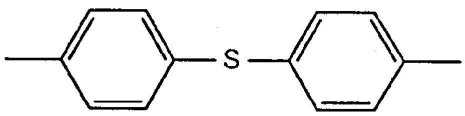 , 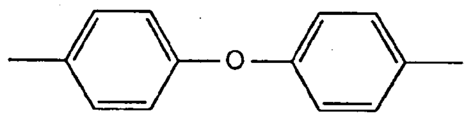 ,

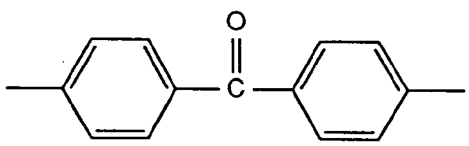 ,

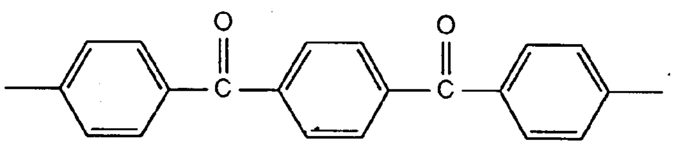

oder

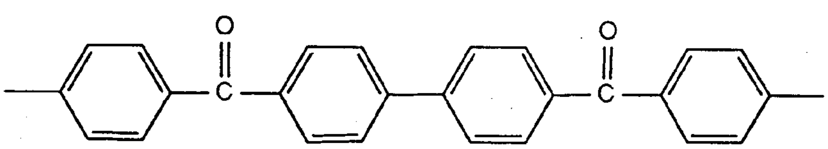

steht.

**10.** Verfahren gemäss Anspruch 7, dadurch gekennzeichnet, dass $Ar_1$ für einen Rest der Formeln

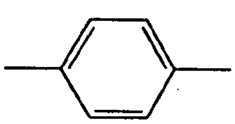 , 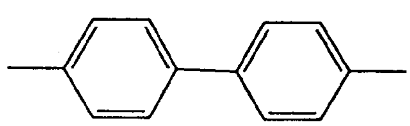

oder

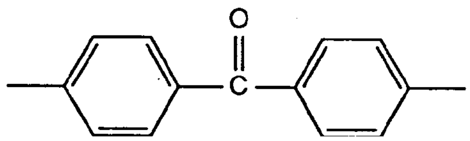

steht.

**11.** Verfahren gemäss Anspruch 7, dadurch gekennzeichnet, dass $Ar_2$ für einen Rest der Formeln

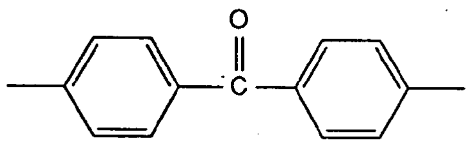

oder

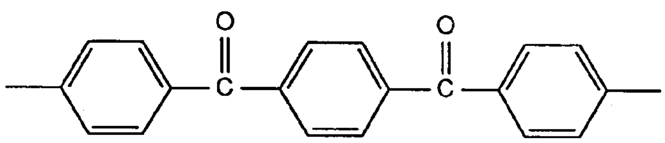

steht.

**12.** Verfahren gemäss Anspruch 7, dadurch gekennzeichnet, dass $Ar_2$ für

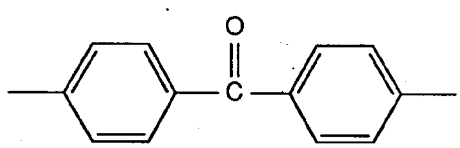

steht.

**13.** Verwendung der nach Anspruch 1 hergestellten Polymeren zur Herstellung von Formkörpern, Folien oder Fasern, als Matrixhaize, Klebstöffe oder Überzugsmittel oder als Zusätze zu anderen Polymeren.

**14.** Verwendung der nach Anspruch 3 hergestellten Polymeren ur Modifizierung von anderen Kunststoffen.

**15.** Zusammensetzung enthaltend
a) mindestens ein Epoxidharz,
b) mindestens einen aminischen oder phenolischen Härter und
c) ein Polymer gemäss Anspruch 4.

**16.** Zusammensetzung gemäss Anspruch 15 enthaltend
a) 20-80 Gew.-% eines Epoxidharzes,
b) 5-50 Gew.-% eines aminischen Härters und
c) 2-40 Gew.-% eines Polymers gemäss Anspruch 4.

**17.** Zusammensetzung gemäss Anspruch 15, enthaltend als Komponente a) ein Epoxidharz ausgewählt aus der Gruppe bestehend aus B isphenol-A-diglycidylether, Bisphenol-F-diglycidylether, Bisphenol-S-diglycidylether, N,N,O-Triglycidyl-p-aminophenol, N,N,O-Triglycidyl-m-aminophenol und N,N,N′,N′-Tetraglycidyldiaminodiphenylmethan und als Komponente b) einen aminischen Härter ausgewählt aus der Gruppe bestehend aus 3,3′-Diaminodiphenylsulfon, 4,4′-Diaminodiphenylsulfon und 4,4-Diaminodiphenylmethan.

Europäisches Patentamt

# EUROPÄISCHER RECHERCHENBERICHT

Nummer der Anmeldung
EP 92810096.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| P,X | CHEMICAL ABSTRACTS, Band 115, Nr. 22, 2. Dezember 1991, Columbus, Ohio, USA J.M. DESIMONE et al. "Thiophene-based thermoplastic poly(arylene ethers)" Seite 47, Zusammenfassung Nr. 233 539r & Polym. Prep. (Am.Chem. Soc.,Div.Polym.Chem.) 1991, 32(2), 172-3 | 1-3,6, 9,15, 17,18 | C 08 G 65/40<br>C 08 L 63/00<br>C 07 D 333/22 |
| A | CHEMICAL ABSTRACTS, Band 96, Nr. 14, 5. April 1982, Columbus, Ohio, USA M.C. VENUTI "2,3-Dihydroxy-1,4-dioxane: a stable synthetic equivalent of anhydrous glyoxal" Seite 701, Zusammenfassung Nr. 104 158f * Synthesis 1982, (1), 61-3 | 17 | |
| | | | RECHERCHIERTE SACHGEBIETE (Int Cl 5) |
| A | CHEMICAL ABSTRACTS, Band 92, Nr. 9, 3. März 1980, Columbus, Ohio, USA Y. MIYAHARA "Facile synthesis of 2,5-diacylthiophenes" Seite 650, Zusammenfassung Nr. 76 204q | 17 | C 08 G 65/00<br>C 08 L 63/00<br>C 07 D 333/00 |
| D | & Heterocycl.Chem. 1979, 16(6), 1147-51 | | |
| D,A | JOURNAL OF POLYMER SCIENCE, TEIL A: POLYMER CHEMISTRY, Band 27, Nr. 7, Juni 1988, New York MI HIEYI et al. "Synthesis and properties of polyketones containing thiophene links" | 1,5 | |

Der vorliegende Recherchenbericht wurde für alle Patentansprüche erstellt.

| Recherchenort | Abschlußdatum der Recherche | Prüfer |
|---|---|---|
| WIEN | 07-05-1992 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 62

Europäisches Patentamt

# EUROPÄISCHER RECHERCHENBERICHT

Nummer der Anmeldung

-2-
EP 92810096.5

| EINSCHLÄGIGE DOKUMENTE | | | |
| --- | --- | --- | --- |
| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
| | Seiten 2417-2426<br>* Gesamt *<br>-- | | |
| D,A | JOURNAL OF POLYMER SCIENCE, TEIL A: POLYMER CHEMISTRY, Band 28, Nr. 11, Oktober 1990, New York<br>KIL-YEONG et al. "Synthesis and properties of polysulfones containing thiophene links"<br>Seiten 3179-3184<br>* Gesamt *<br>---- | 1 | |
| | | | RECHERCHIERTE SACHGEBIETE (Int. Cl 5) |

Der vorliegende Recherchenbericht wurde für alle Patentansprüche erstellt.

| Recherchenort | Abschlußdatum der Recherche | Prüfer |
| --- | --- | --- |
| WIEN | 07-05-1992 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82